# EUROPEAN PATENT SPECIFICATION

(11) **EP 3 932 323 B1**
(45) Date of publication and mention of the grant of the patent: **04.09.2024**
(21) Application number: 19917336.0
(22) Date of filing: 20.11.2019
(51) Int. Cl.: A61B 8/12, A61B 1/00, A61B 1/045, A61B 8/00

(54) **ULTRASONIC ENDOSCOPIC SYSTEM AND OPERATING METHOD OF ULTRASONIC ENDOSCOPIC SYSTEM**
ULTRASCHALL-ENDOSKOPIESYSTEM UND BETRIEBSVERFAHREN FÜR EIN ULTRASCHALL-ENDOSKOPIESYSTEM
SYSTÈME ENDOSCOPIQUE À ULTRASONS ET PROCÉDÉ DE FONCTIONNEMENT DE SYSTÈME ENDOSCOPIQUE À ULTRASONS

(30) Priority: 28.02.2019 JP 2019035846; 29.08.2019 JP 2019156614
(43) Date of publication of application: 05.01.2022
(73) Proprietor: FUJIFILM Corporation, Tokyo 106-8620 (JP)
(72) Inventor: UCHIHARA Masanobu, Kanagawa 258-8538 (JP); TANAKA Toshizumi, Kanagawa 258-8538 (JP); MORIMOTO Yasuhiko, Kanagawa 258-8538 (JP)
(74) Representative: Kudlek, Franz Thomas
(86) International application number: PCT/JP2019/045429
(87) International publication number: WO 2020/174778

(56) References cited:
- WO-A1-2017/195540
- WO-A1-2018/225448
- WO-A1-2018/225448
- JP-A- 2011 206 428
- JP-A- 2011 206 428
- JP-A- 2017 202 125

## Description

### BACKGROUND OF THE INVENTION

### 1. Field of the Invention

The present invention relates to an ultrasound endoscope system and a method of operating an ultrasound endoscope system that observe a state of an observation target part in a body of a subject using an ultrasonic wave.

### 2. Description of the Related Art

For example, in an ultrasound endoscope system, for the primary purpose of observation of pancreas, a gallbladder, or the like using a trans-digestive tract, an ultrasound endoscope having an endoscope observation part and an ultrasound observation part at a distal end is inserted into a digestive tract of a subject, and an endoscope image inside the digestive tract and an ultrasound image of a part outside a wall of the digestive tract are captured.

In the ultrasound endoscope system, an observation target adjacent part inside the digestive tract is irradiated with illumination light from an illumination part provided at the distal end of the ultrasound endoscope, reflected light of illumination light is received by an imaging part provided at the distal end of the ultrasound endoscope, and an endoscope image is generated from an imaging signal of reflected light. Ultrasonic waves are transmitted and received to and from an observation target part, such as an organ outside the wall of the digestive tract, by a plurality of ultrasound transducers provided at the distal end of the ultrasound endoscope, and an ultrasound image is generated from reception signals of the ultrasonic waves.

In a case of observing the endoscope image, an operator (a user of the ultrasound endoscope system) comparatively easily knows a position of the distal end portion of the ultrasound endoscope in a digestive organ of the subject, a direction of the distal end portion of the ultrasound endoscope, and a part being observed at this moment in such a manner that, in a case where the ultrasound endoscope is inserted into the digestive tract of the subject, for example, an inner wall of an esophagus comes into view, and in a case where a distal end portion of the ultrasound endoscope is further pushed forward, an inner wall of a stomach comes into view.

In contrast, there is a problem in that an operator who is unaccustomed to an ultrasound image extremely hardly knows what is displayed in the ultrasound image, for example, pancreas or a gallbladder or a blood vessel, a bile duct, or a pancreatic duct, and a range of an organ, such as pancreas or a gallbladder, displayed in the ultrasound image. There is also a problem in that the operator who is unaccustomed to an ultrasound image does not know a position of the distal end portion of the ultrasound endoscope, a direction of the distal end portion of the ultrasound endoscope, and a part being observed at this moment during the observation of the ultrasound image, and gets lost in the body of the subject.

Here, examples of the related art having relation to the invention are JP1994-233761A (JP-H06-233761A), JP2010-069018A, JP1990-045045A (JP-H02-045045A), and JP2004-113629A.

JP1994-233761A (JP-H06-233761A) describes that an intended part in an image of a diagnosis part inside a subject is roughly extracted, global information for recognizing the intended part is predicted using a neural network, a contour of the intended part is recognized using the global information, and a recognition result is displayed along with an original image.

JP2010-069018A describes that position and alignment data of a distal end portion of an ultrasound endoscope is generated based on an electric signal from a coil, insertion shape data for indicating an insertion shape of the ultrasound endoscope is generated from the position and alignment data, a guide image is generated by combining the insertion shape data with three-dimensional biological tissue model data of a tissue structure of an organ group or the like of a subject, and a video signal of a composite image, in which an ultrasound image and the guide image are composed, is generated and displayed on a monitor.

JP2010-069018A describes that the composite image is displayed such that a stereoscopic guide image and a cross-sectional guide image are disposed in a left region of a screen, and the ultrasound image is disposed in a right region of the screen.

JP2010-069018A describes a button for enlarging or reducing a display range of the ultrasound image.

JP1990-045045A (JP-H02-045045A) describes that an ultrasonic tomographic image and an optical image of a subject are displayed at one place adjacently within a screen of a display device to observe such that both images can be observed simultaneously.

JP2004-113629A describes that an ultrasound image and a schematic view are displayed on the same screen, the schematic view is a schema diagram or an actual optical image of a human body, and a scanning plane and an insertion shape of an ultrasound endoscope are displayed together in the schematic view.

JP2004-113629A describes that a region of a scanning position of the ultrasound endoscope is detected from a signal of a position and a direction of the ultrasound endoscope detected using a coil to output ultrasound scanning region data, part name data corresponding to the ultrasound scanning region data is read from a part name storage unit, and a part name is superimposedly displayed on an ultrasound image.

Further, document JP 2011 206428 A for example provides an intracoelomic insertion type ultrasonic examination device to elongate a curved part in order to gently bend a connection pipe and to prevent the interference with another member arranged in an insertion part. The connection pipe, of which leading end part is inserted in and fitted to the treatment device insertion passage formed to the leading end body constituting a leading end hard part, is formed into a curved shape and extended into the leading end ring constituting the curved part in a backward warped state to be backward warped from the state almost coming into contact with the inner surface of the leading end ring. A treatment device insertion tube is connected to the upward warped region of the connection pipe and a cutting squeeze part, to which the leading end of a curving operation wire is fastened, is provided to the lower position of the upward warped region.

JP 2017 202125 A for example refers to an ultrasonic imaging device including an image generation part for transmitting an ultrasonic wave to a subject, generating an ultrasonic 2D image from a reception signal of an ultrasonic probe for receiving the ultrasonic wave from the subject, and generating an ultrasonic 3D image by transmitting/receiving the ultrasonic wave a plurality of times, and an image processing device for processing the ultrasonic 2D image and the ultrasonic 3D image. The image processing device includes an ultrasonic 3D image feature region position estimation/identification part for estimating/identifying a feature region of the subject from the ultrasonic 3D image acquired by an ultrasonic 3D image acquisition part, an ultrasonic 2D-3D image positioning part for positioning the ultrasonic 2D image and the ultrasonic 3D image, and an image display part for displaying information on the position, the name, and a distance relationship with the ultrasonic 2D image of the feature region on a real time ultrasonic 2D image using the acquired position information on the feature region and the positioning result.

WO 2018/225448 A1 for example provides a disease diagnosis support method and the like which employ an endoscopic image of a digestive organ, and in which a neural network is used. This disease diagnosis support method, which employs an endoscopic image of a digestive organ, and in which a neural network is used, is characterized in that the neural network is trained using a first endoscopic image of a digestive organ, and, corresponding to the first endoscopic image, a confirmed diagnosis result of at least one of a positive or negative diagnosis of the disease of said digestive organ, previous disease, a level of severity, or information corresponding to the imaged site, and on the basis of a second endoscopic image of a digestive organ, the trained neural network outputs at least one of the probability of a positive and/or negative diagnosis of the disease of said digestive organ, the probability of previous disease, the level of severity of the disease, and information corresponding to the imaged site.

### SUMMARY OF THE INVENTION

JP1994-233761A (JP-H06-233761A) describes that a contour of an intended part in an image of a diagnosis part is recognized, but does not describe that a name of the intended part is recognized from the image.

JP2010-069018A and JP2004-113629A describe that the position and the orientation of the distal end portion of the ultrasound endoscope are detected using the coil, but does not describe that the position and the orientation of the distal end portion of the ultrasound endoscope are detected from the ultrasound image without needing an additional component, such as the coil.

JP2010-069018A, JP1990-045045A (JP-H02-045045A), and JP2004-113629A describe that the guide image, such as a schema diagram, the endoscope image, and the ultrasound image are displayed in combination, but does not describe that a combination of the images is switched and displayed easily to see in response to an instruction from a user. Furthermore, there is no description that the guide image, such as a schema diagram, the endoscope image, and the ultrasound image are displayed in combination.

JP2004-113629A describes that the part name is superimposedly displayed on the ultrasound image, but does not describe that the name of the part displayed in the ultrasound image is recognized from the ultrasound image without using additional data, such as part name data, and displayed.

JP1994-233761A (JP-H06-233761A), JP2010-069018A, JP1990-045045A (JP-H02-045045A), and JP2004-113629A do not describe that the name of the organ and the range of the organ displayed in the ultrasound image are displayed simultaneously, the position and the orientation of the distal end portion of the ultrasound endoscope and the range of the organ are displayed simultaneously, or the name of the organ displayed in the ultrasound image, the position and the orientation of the distal end portion of the ultrasound endoscope, and the range of the organ displayed in the ultrasound image are displayed simultaneously.

Accordingly, a first object of the invention is to provide an ultrasound endoscope system and a method of operating an ultrasound endoscope system capable of recognizing a name of an organ displayed in an ultrasound image, a range of the organ, and a position and an orientation of a distal end portion of an ultrasound endoscope from the ultrasound image, and displaying the recognized information on a monitor.

A second object of the invention is to provide an ultrasound endoscope system and a method of operating an ultrasound endoscope system capable of switching and displaying an endoscope image, an ultrasound image, and an anatomical schema diagram easily to see in response to an instruction from a user.

The present invention relates to an ultrasound endoscope system and a method of operating an ultrasound endoscope system with the features of the independent claims. To achieve the above-described objects, the invention provides an ultrasound endoscope system comprising an ultrasound endoscope that has an ultrasound transducer at a distal end, an ultrasound observation device that makes the ultrasound transducer transmit and receive an ultrasonic wave and generates an ultrasound image for diagnosis from a reception signal of the ultrasonic wave, an ultrasound image recognition unit that learns at least one of a relationship between an ultrasound image for learning and a name of an organ displayed in the ultrasound image for learning or a relationship between the ultrasound image for learning and a position of a distal end portion of the ultrasound endoscope at the time of imaging of the ultrasound image for learning, on a plurality of the ultrasound images for learning in advance, and recognizes at least one of a name of an organ displayed in the ultrasound image for diagnosis or a position of the distal end portion of the ultrasound endoscope from the ultrasound image for diagnosis based on a learning result, and a display controller that displays at least one of the name of the organ or the position of the distal end portion of the ultrasound endoscope recognized by the ultrasound image recognition unit, on a monitor.

The ultrasound endoscope system further comprises an instruction acquisition unit that acquires an instruction input from a user, and in response to an instruction from the user, the display controller superimposedly displays the name of the organ on the ultrasound image for diagnosis and superimposedly displays the position of the distal end portion of the ultrasound endoscope on an anatomical schema diagram.

In response to an instruction from the user, the display controller displays two or more images including at least one of the ultrasound image for diagnosis with the name of the organ superimposedly displayed or the anatomical schema diagram with the position of the distal end portion of the ultrasound endoscope superimposedly displayed from among the ultrasound image for diagnosis with the name of the organ not displayed, the ultrasound image for diagnosis with the name of the organ superimposedly displayed, the anatomical schema diagram with the position of the distal end portion of the ultrasound endoscope not displayed, and the anatomical schema diagram with the position of the distal end portion of the ultrasound endoscope superimposedly displayed, in parallel within a screen of the monitor.

It is preferable that the ultrasound endoscope further has an illumination part and an imaging part at the distal end, the ultrasound endoscope system further comprises an endoscope processor that makes the imaging part receive reflected light of illumination light emitted from the illumination part and generates an endoscope image for diagnosis from an imaging signal of the reflected light, and an instruction acquisition unit that acquires an instruction input from a user, and the display controller displays the endoscope image for diagnosis within a screen of the monitor in response to an instruction from the user.

It is preferable that the ultrasound endoscope system further comprises an endoscope image recognition unit that recognizes a lesion region displayed in the endoscope image for diagnosis from the endoscope image for diagnosis, and in response to an instruction from the user, the display controller displays the endoscope image for diagnosis with the lesion region superimposedly displayed, on the monitor.

It is preferable that, in response to an instruction from the user, the display controller displays two or more images including at least one of the ultrasound image for diagnosis with the name of the organ superimposedly displayed or an anatomical schema diagram with the position of the distal end portion of the ultrasound endoscope superimposedly displayed from among the endoscope image for diagnosis with the lesion region not displayed, the endoscope image for diagnosis with the lesion region superimposedly displayed, the ultrasound image for diagnosis with the name of the organ not displayed, the ultrasound image for diagnosis with the name of the organ superimposedly displayed, an anatomical schema diagram with the position of the distal end portion of the ultrasound endoscope not displayed, and an anatomical schema diagram with the position of the distal end portion of the ultrasound endoscope superimposedly displayed, in parallel within the screen of the monitor.

It is preferable that one image of the two or more images displayed on the monitor is displayed as an image of interest to be greater than other images.

It is preferable that the display controller switches and displays the image of interest from the one image to one of the other images in response to an instruction from the user.

It is preferable that, in response to an instruction from the user, the display controller displays the ultrasound image for diagnosis with the name of the organ not displayed, the ultrasound image for diagnosis with the name of the organ superimposedly displayed, and the anatomical schema diagram with the position of the distal end portion of the ultrasound endoscope superimposedly displayed, in parallel within the screen of the monitor.

It is preferable that the ultrasound image recognition unit operates in a case where the ultrasound image for diagnosis or an anatomical schema diagram is displayed within the screen of the monitor, and the endoscope image recognition unit operates in a case where the endoscope image for diagnosis is displayed within the screen of the monitor.

It is preferable that the ultrasound image recognition unit learns at least one of the relationship between the ultrasound image for learning and the name of the organ displayed in the ultrasound image for learning or a relationship between the ultrasound image for learning and the position and an orientation of the distal end portion of the ultrasound endoscope at the time of imaging of the ultrasound image for learning, on the plurality of ultrasound images for learning in advance, and recognizes at least one of the name of the organ displayed in the ultrasound image for diagnosis or the position and an orientation of the distal end portion of the ultrasound endoscope from the ultrasound image for diagnosis based on a learning result, and the display controller displays at least one of the name of the organ recognized by the ultrasound image recognition unit or the position and the orientation of the distal end portion of the ultrasound endoscope recognized by the ultrasound image recognition unit, on the monitor.

It is preferable that the ultrasound endoscope system further comprises an instruction acquisition unit that acquires an instruction input from a user, and in response to an instruction from the user, the display controller displays an anatomical schema diagram with the position and the orientation of the distal end portion of the ultrasound endoscope superimposedly displayed, on the monitor.

It is preferable that the ultrasound image recognition unit further learns a relationship between the ultrasound image for learning and a range of the organ displayed in the ultrasound image for learning, on the plurality of ultrasound images for learning in advance, and recognizes the range of the organ displayed in the ultrasound image for diagnosis from the ultrasound image for diagnosis based on a learning result, and the display controller further displays the range of the organ recognized by the ultrasound image recognition unit, on the monitor.

It is preferable that the display controller colors an internal region of the range of the organ recognized by the ultrasound image recognition unit and displays the range of the organ with the internal region colored, on the monitor or provides a frame indicating the range of the organ recognized by the ultrasound image recognition unit, colors the frame, and displays the range of the organ with the frame colored, on the monitor.

It is preferable that the display controller colors the internal region or the frame in a different color for each type of organ with the range recognized by the ultrasound image recognition unit.

It is preferable that the ultrasound endoscope system further comprises an instruction acquisition unit that acquires an instruction input from a user, and a color registration unit that registers a relationship between the type of the organ and the color of the internal region or the frame in response to an instruction from the user, and the display controller colors the internal region or the frame in a color designated by the instruction from the user or colors the internal region or the frame in a color of the internal region or the frame corresponding to the type of the organ registered in the color registration unit.

It is preferable that the ultrasound image recognition unit further calculates a confidence factor of the name of the organ recognized by the ultrasound image recognition unit, and the display controller decides at least one of a display method of the name of the organ displayed on the monitor or a coloring method of the internal region or the frame depending on the confidence factor.

It is preferable that, in a case of superimposedly displaying the name of the organ on the ultrasound image for diagnosis, the display controller decides at least one of the color of the name of the organ or the color of the internal region or the frame depending on brightness of the ultrasound image for diagnosis displayed behind a display region of the name of the organ.

It is preferable that the ultrasound endoscope system further comprises an instruction acquisition unit that acquires an instruction input from a user, and the display controller switches whether to display only one, both, or none of the name of the organ recognized by the ultrasound image recognition unit and the range of the organ with the internal region or the frame colored, in response to an instruction from the user.

It is preferable that the display controller decides a position where the name of the organ recognized by the ultrasound image recognition unit is displayed on the monitor, depending on the range of the organ recognized by the ultrasound image recognition unit.

It is preferable that the display controller decides whether or not to display the name of the organ recognized by the ultrasound image recognition unit on the monitor, depending on the range of the organ recognized by the ultrasound image recognition unit.

It is preferable that the ultrasound endoscope system further comprises an instruction acquisition unit that acquires an instruction input from a user, and an organ registration unit that registers a type of an organ for displaying a range in response to an instruction from the user, and in a case where an organ with a range recognized by the ultrasound image recognition unit is an organ registered in the organ registration unit, the display controller displays the range of the organ recognized by the ultrasound image recognition unit, on the monitor.

It is preferable that the ultrasound endoscope system further comprises an instruction acquisition unit that acquires an instruction input from a user, and the display controller sequentially switches a type of an organ for displaying a range in response to an instruction from the user.

It is preferable that the ultrasound image recognition unit is incorporated in the ultrasound observation device.

It is preferable that the ultrasound endoscope further has an illumination part and an imaging part at the distal end, the ultrasound endoscope system further comprises an endoscope processor that makes the imaging part receive reflected light of illumination light emitted from the illumination part and generates an endoscope image for diagnosis from an imaging signal of the reflected light, and the ultrasound image recognition unit is incorporated in the endoscope processor.

It is preferable that the ultrasound endoscope further has an illumination part and an imaging part at the distal end, the ultrasound endoscope system further comprises an endoscope processor that makes the imaging part receive reflected light of illumination light emitted from the illumination part and generates an endoscope image for diagnosis from an imaging signal of the reflected light, and the ultrasound image recognition unit is provided outside the ultrasound observation device and the endoscope processor.

The invention provides a method of operating an ultrasound endoscope system, the method comprising a step of, with an ultrasound image recognition unit, learning at least one of a relationship between an ultrasound image for learning and a name of an organ displayed in the ultrasound image for learning or a relationship between the ultrasound image for learning and a position of a distal end portion of an ultrasound endoscope at the time of imaging of the ultrasound image for learning, on a plurality of the ultrasound images for learning in advance, a step of, with an ultrasound observation device, making an ultrasound transducer provided at a distal end of the ultrasound endoscope transmit and receive an ultrasonic wave and generates an ultrasound image for diagnosis from a reception signal of the ultrasonic wave, a step of, with the ultrasound image recognition unit, recognizing at least one of a name of an organ displayed in the ultrasound image for diagnosis or a position of the distal end portion of the ultrasound endoscope from the ultrasound image for diagnosis based on a learning result, and a step of, with a display controller, displaying at least one of the name of the organ or the position of the distal end portion of the ultrasound endoscope recognized by the ultrasound image recognition unit, on a monitor.

In response to an instruction from a user, the name of the organ is superimposedly displayed on the ultrasound image for diagnosis, and the position of the distal end portion of the ultrasound endoscope is superimposedly displayed on an anatomical schema diagram.

In response to an instruction from the user, two or more images including at least one of the ultrasound image for diagnosis with the name of the organ superimposedly displayed or the anatomical schema diagram with the position of the distal end portion of the ultrasound endoscope superimposedly displayed from among the ultrasound image for diagnosis with the name of the organ not displayed, the ultrasound image for diagnosis with the name of the organ superimposedly displayed, the anatomical schema diagram with the position of the distal end portion of the ultrasound endoscope not displayed, and the anatomical schema diagram with the position of the distal end portion of the ultrasound endoscope superimposedly displayed are displayed in parallel within a screen of the monitor.

It is preferable that one image of the two or more images displayed on the monitor is displayed as an image of interest to be greater than other images.

It is preferable that the image of interest is switched from the one image to one of the other images and displayed in response to an instruction from the user.

It is preferable that at least one of the relationship between the ultrasound image for learning and the name of the organ displayed in the ultrasound image for learning or a relationship between the ultrasound image for learning and the position and orientation of the distal end portion of the ultrasound endoscope at the time of imaging of the ultrasound image for learning is learned on the plurality of ultrasound images for learning in advance,
at least one of the name of the organ displayed in the ultrasound image for diagnosis or the position and the orientation of the distal end portion of the ultrasound endoscope is recognized from the ultrasound image for diagnosis based on a learning result, and at least one of the name of the organ recognized by the ultrasound image recognition unit or the position and the orientation of the distal end portion of the ultrasound endoscope recognized by the ultrasound image recognition unit is displayed on the monitor.

It is preferable that the method further comprises a step of, with the ultrasound image recognition unit, learning a relationship between the ultrasound image for learning and a range of the organ displayed in the ultrasound image for learning, on the plurality of ultrasound images for learning in advance, a step of, with the ultrasound image recognition unit, recognizing the range of the organ displayed in the ultrasound image for diagnosis from the ultrasound image for diagnosis based on a learning result, and a step of, with the display controller, further displaying the range of the organ recognized by the ultrasound image recognition unit, on the monitor.

It is preferable that the ultrasound image recognition unit, the display controller, the instruction acquisition unit, and the endoscope image recognition unit are hardware or a processor that executes a program, and it is preferable that the color registration unit and the organ registration unit are hardware or a memory.

According to the invention, since the name of the organ displayed in the ultrasound image for diagnosis and the range of the organ are displayed on the monitor, for example, even a user who is unaccustomed to an ultrasound image can correctly recognize what is displayed in the ultrasound image and the range of the organ displayed ultrasound image. Furthermore, since the position and the orientation of the distal end portion of the ultrasound endoscope are displayed on the monitor, for example, even a user who is unaccustomed to an ultrasound image can correctly recognize a position of the distal end portion of the ultrasound endoscope, a direction of the distal end portion of the ultrasound endoscope, and a part being observed at this moment, and does not get lost in a body of a subject.

According to the invention, it is possible to switch and display an endoscope image, an ultrasound image, and an anatomical schema diagram easily to see. While an image of interest in which the user is interested changes occasionally, the user can switch the image of interest at any timing, and thus, it is possible to allow the user to display and view an image in which the user is interested on the occasion, as an image of interest to be greater than other images.

### BRIEF DESCRIPTION OF THE DRAWINGS

Fig. 1 is a diagram showing the schematic configuration of an ultrasound endoscope system according to a first embodiment of the invention.
Fig. 2 is a plan view showing a distal end portion of an insertion part of an ultrasound endoscope and the periphery of the distal end portion.
Fig. 3 is a sectional view showing a cross section of the distal end portion of the insertion part of the ultrasound endoscope taken along the line I-I of Fig. 2.
Fig. 4 is a block diagram of an embodiment representing the configuration of an endoscope image recognition unit.
Fig. 5 is a block diagram showing the configuration of an ultrasound observation device.
Fig. 6 is a block diagram of an embodiment representing the configuration of an ultrasound image recognition unit.
Fig. 7A is an anatomical schema diagram of an embodiment representing a confluence of an aorta, a celiac artery, and a superior mesenteric artery.
Fig. 7B is a conceptual diagram of an embodiment representing an ultrasound image of the confluence of the aorta, the celiac artery, and the superior mesenteric artery shown in Fig. 7A.
Fig. 8A is an anatomical schema diagram of an embodiment representing a pancreatic tail.
Fig. 8B is a conceptual diagram of an embodiment representing an ultrasound image of the pancreatic tail shown in Fig. 8A.
Fig. 9A is an anatomical schema diagram of an embodiment representing a confluence of a splenic vein, a superior mesenteric vein, and a portal vein.
Fig. 9B is a conceptual diagram of an embodiment representing an ultrasound image of the confluence of the splenic vein, the superior mesenteric vein, and the portal vein shown in Fig. 9A.
Fig. 10A is an anatomical schema diagram of an embodiment representing a pancreatic head.
Fig. 10B is a conceptual diagram of an embodiment representing an ultrasound image of the pancreatic head shown in Fig. 10A.
Fig. 11 is a flowchart showing a flow of diagnosis processing using the ultrasound endoscope system.
Fig. 12 is a flowchart showing a procedure of a diagnosis step during the diagnosis processing.
Fig. 13A is a conceptual diagram of an embodiment representing display positions of an ultrasound image for diagnosis and an anatomical schema diagram.
Fig. 13B is a conceptual diagram of an embodiment representing the ultrasound image for diagnosis and the anatomical schema diagram shown in Fig. 13A.
Fig. 14A is a conceptual diagram of an embodiment representing display positions of a first ultrasound image for diagnosis, a second ultrasound image for diagnosis, and an anatomical schema diagram.
Fig. 14B is a conceptual diagram of an embodiment representing the first ultrasound image for diagnosis, the second ultrasound image for diagnosis, and the anatomical schema diagram shown in Fig. 14A.
Fig. 15A is a conceptual diagram of an embodiment representing display positions of an endoscope image, an ultrasound image for diagnosis, and an anatomical schema diagram.
Fig. 15B is a conceptual diagram of an embodiment representing the endoscope image, the ultrasound image for diagnosis, and the anatomical schema diagram shown in Fig. 15A.
Fig. 16 is a conceptual diagram of an embodiment representing a manner in which an image of interest is switched from one image of images displayed on a monitor to one of other images and displayed.
Fig. 17 is a block diagram of an embodiment representing an ultrasound endoscope system in a case where an ultrasound image recognition unit is incorporated in an ultrasound observation device.
Fig. 18 is a block diagram of an embodiment representing the configuration of an ultrasound endoscope system in a case where an ultrasound image recognition unit is incorporated in an endoscope processor.
Fig. 19 is a block diagram of an embodiment representing the configuration of an ultrasound endoscope system in a case where an ultrasound image recognition unit is provided outside an ultrasound observation device and an endoscope processor.
Fig. 20 is a block diagram showing the configuration of an ultrasound observation device of a second embodiment.
Fig. 21 is a block diagram showing the configuration of an ultrasound image recognition unit of the second embodiment.
Fig. 22A is a conceptual diagram of an embodiment representing an ultrasound image in which only a name of an organ is displayed without coloring a range of the organ.
Fig. 22B is a conceptual diagram of an embodiment representing an ultrasound image in which only the range of an organ is colored without displaying the name of the organ.

### DESCRIPTION OF THE PREFERRED EMBODIMENTS

An ultrasound endoscope system according to an embodiment (the embodiment) of the invention will be described below in detail referring to preferred embodiments shown in the accompanying drawings.

The embodiment is a representative embodiment of the invention, but is merely an example and does not limit the invention.

### <<Outline of Ultrasound Endoscope System>>

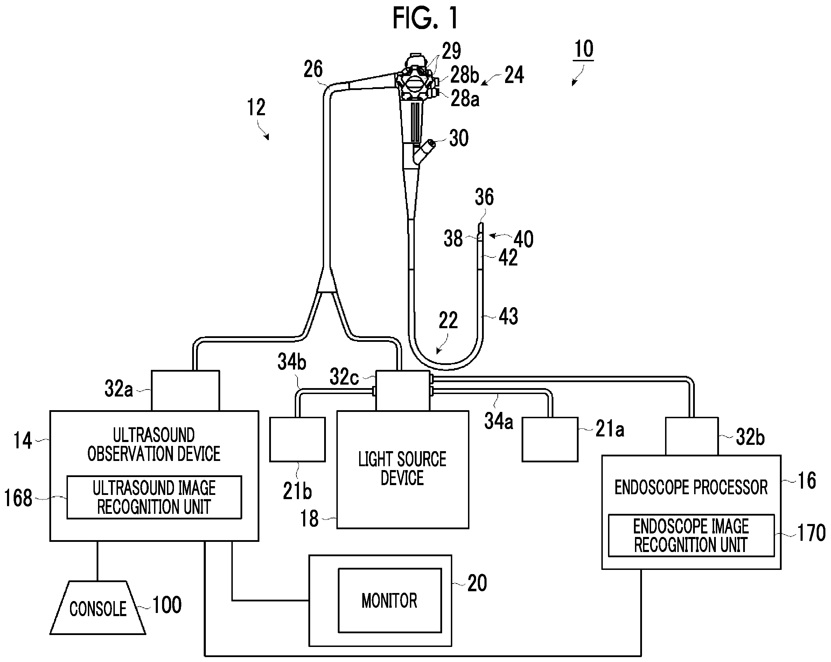

The outline of an ultrasound endoscope system 10 according to the embodiment will be described referring to Fig. 1. Fig. 1 is a diagram of a first embodiment showing the schematic configuration of an ultrasound endoscope system 10.

The ultrasound endoscope system 10 is used to make observation (hereinafter, referred to as ultrasonography) a state of an observation target part in a body of a patient as a subject using ultrasonic waves. Here, the observation target part is a part that is hardly examined from a body surface side of the patient, and is, for example, pancreas or a gallbladder. With the use of the ultrasound endoscope system 10, it is possible to perform ultrasonography of a state of the observation target part and the presence or absence of an abnormality by way of digestive tracts, such as esophagus, stomach, duodenum, small intestine, and large intestine, which are body cavities of the patient.

The ultrasound endoscope system 10 acquires an ultrasound image and an endoscope image, and as shown in Fig. 1, as an ultrasound endoscope 12, an ultrasound observation device 14, an endoscope processor 16, a light source device 18, a monitor 20, a water supply tank 21a, a suction pump 21b, and a console 100.

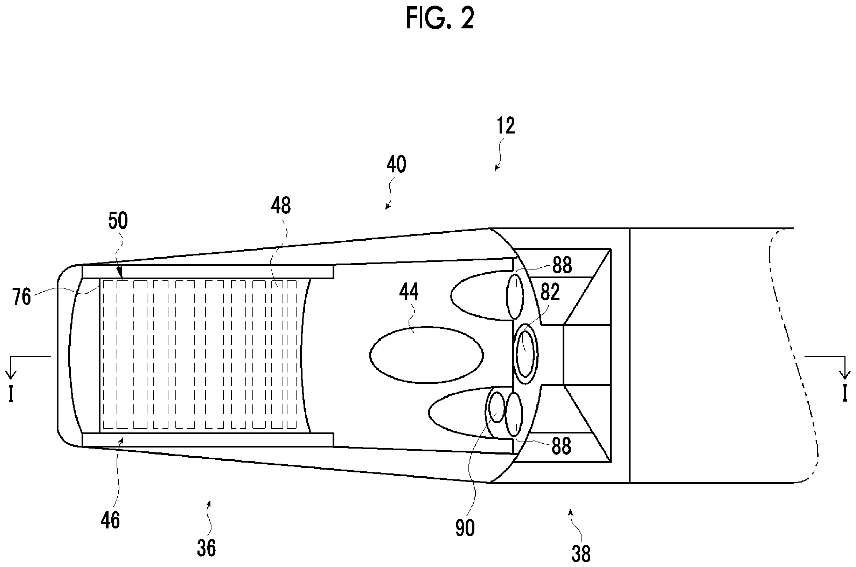

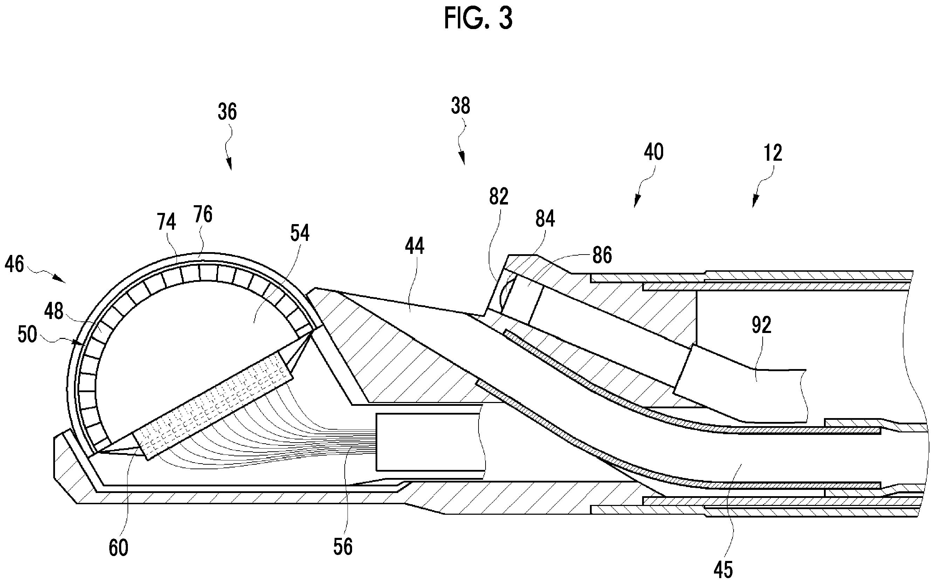

The ultrasound endoscope 12 comprises an insertion part 22 that is inserted into the body cavity of the patient, an operating part 24 that is operated by an operator (user), such as a physician or a technician, and an ultrasound transducer unit 46 (see Figs. 2 and 3) that is attached to a distal end portion 40 of the insertion part 22. The ultrasound endoscope 12 has a plurality of ultrasound transducers 48 of an ultrasound transducer unit 46 as an ultrasound observation part 36 at the distal end (see Figs. 2 and 3). Furthermore, the ultrasound endoscope 12 has an illumination part including illumination windows 88 and the like and an imaging part including an observation window 82, an objective lens 84, a solid-state imaging element 86, and the like as an endoscope observation part 38 at the distal end (see Figs. 2 and 3). The operator acquires an endoscope image and an ultrasound image by the function of the ultrasound endoscope 12.

Here, the "endoscope image" is an image that is obtained by imaging a body cavity inner wall of the patient using an optical method. Furthermore, the "ultrasound image" is an image that is obtained by receiving reflected waves (echoes) of ultrasonic waves transmitted from the inside of the body cavity of the patient toward the observation target part and imaging reception signals.

The ultrasound endoscope 12 will be described below in detail.

The ultrasound observation device 14 is connected to the ultrasound endoscope 12 through a universal cord 26 and an ultrasound connector 32a provided in an end portion of the universal cord 26. The ultrasound observation device 14 performs control such that the ultrasound transducer unit 46 of the ultrasound endoscope 12 transmits the ultrasonic waves. The ultrasound observation device 14 generates the ultrasound image by imaging the reception signals when the ultrasound transducer unit 46 receives the reflected waves (echoes) of the transmitted ultrasonic waves. In other words, the ultrasound observation device 14 makes a plurality of ultrasound transducers 48 of the ultrasound transducer unit 46 transmit and receive ultrasonic waves and generates an ultrasound image for diagnosis (hereinafter, simply referred to as an ultrasound image) from reception signals of the ultrasonic waves.

The ultrasound observation device 14 will be described below in detail.

The endoscope processor 16 is connected to the ultrasound endoscope 12 through the universal cord 26 and an endoscope connector 32b provided in the end portion of the universal cord 26. The endoscope processor 16 acquires image data of an observation target adjacent part imaged by the ultrasound endoscope 12 (in detail, the solid-state imaging element 86 described below) and executes predetermined image processing on the acquired image data to generate an endoscope image. In other words, the endoscope processor 16 makes the imaging part provided at the distal end of the ultrasound endoscope 12 receive reflected light of illumination light emitted from the illumination part provided at the distal end of the ultrasound endoscope 12 similarly and generates an endoscope image for diagnosis (hereinafter, simply referred to as an endoscope image) from an imaging signal of the reflected light.

Here, the "observation target adjacent part" is a portion that is at a position adjacent to the observation target part in the inner wall of the body cavity of the patient.

In the embodiment, the ultrasound observation device 14 and the endoscope processor 16 are configured with two devices (computers) provided separately. However, the invention is not limited thereto, and both of the ultrasound observation device 14 and the endoscope processor 16 may be configured with one device.

The light source device 18 is connected to the ultrasound endoscope 12 through the universal cord 26 and a light source connector 32c provided in the end portion of the universal cord 26. The light source device 18 irradiates the observation target adjacent part with white light composed of three primary color light of red light, green light, and blue light or light having a specific wavelength in imaging the observation target adjacent part using the ultrasound endoscope 12. Light emitted from the light source device 18 propagates through the ultrasound endoscope 12 through a light guide (not shown) included in the universal cord 26 and is emitted from the ultrasound endoscope 12 (in detail, the illumination windows 88 described below). With this, the observation target adjacent part is illuminated with light from the light source device 18.

The monitor 20 is connected to the ultrasound observation device 14 and the endoscope processor 16, and displays an anatomical schema diagram and the like in addition to an ultrasound image generated by the ultrasound observation device 14 and an endoscope image generated by the endoscope processor 16.

As a display method of the ultrasound image and the endoscope image, a method in which one image is switched to one of other images and displayed on the monitor 20 or a method in which two or more images are simultaneously arranged and displayed may be applied.

In the embodiment, although the ultrasound image and the endoscope image are displayed on one monitor 20, a monitor for ultrasound image display, a monitor for endoscope image display, and a monitor for an anatomical schema diagram may be provided separately. Alternatively, the ultrasound image and the endoscope image may be displayed in a display form other than the monitor 20, for example, in a form of being displayed on a display of a terminal carried with the operator.

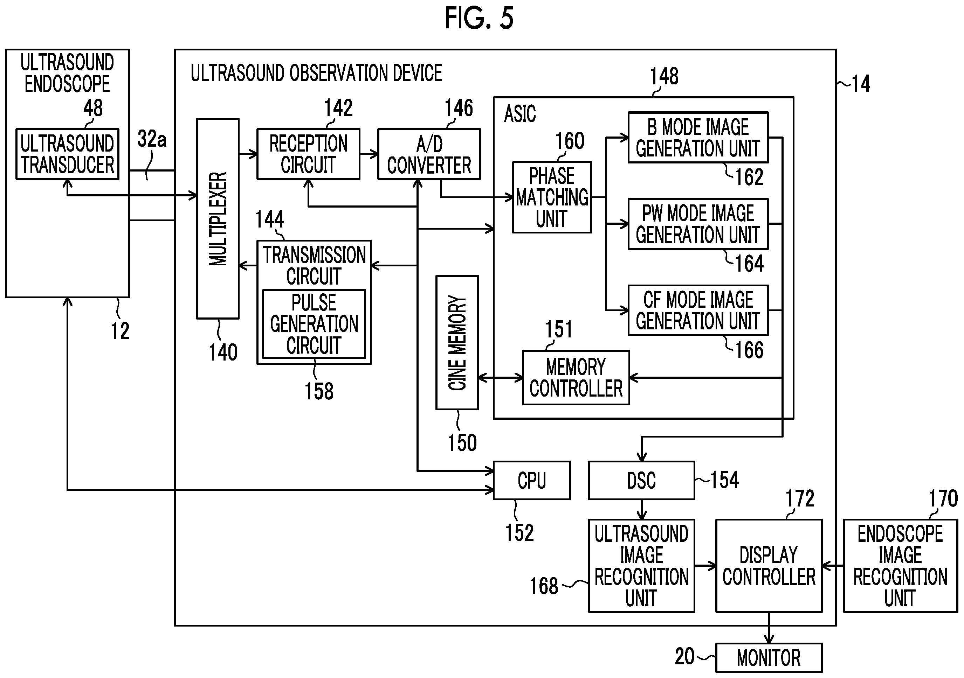

The console 100 is an example of an instruction acquisition unit that acquires an instruction input from the operator (user), and is a device that is provided to allow the operator to input necessary information in a case of ultrasonography, to issue an instruction to start ultrasonography to the ultrasound observation device 14, and the like. The console 100 is configured with, for example, a keyboard, a mouse, a trackball, a touch pad, a touch panel, and the like. In a case where the console 100 is operated, a CPU (control circuit) 152 (see Fig. 5) of the ultrasound observation device 14 controls respective units (for example, a reception circuit 142 and a transmission circuit 144 described below) of the device according to the operation content.

Specifically, the operator inputs examination information (for example, examination order information including date, an order number, and the like and patient information including a patient ID, a patient name, and the like) through the console 100 in a state before starting ultrasonography. After the input of the examination information is completed, in a case where the operator issues an instruction to start ultrasonography through the console 100, the CPU 152 of the ultrasound observation device 14 controls the respective units of the ultrasound observation device 14 such that ultrasonography is executed based on the input examination information.

Furthermore, the operator can set various control parameters through the console 100 in executing ultrasonography. As the control parameters, for example, a selection result of a live mode and a freeze mode, a set value of a display depth (depth), a selection result of an ultrasound image generation mode, and the like are exemplified.

Here, the "live mode" is a mode where ultrasound images (video) obtained at a predetermined frame rate are displayed successively (displayed in real time). The "freeze mode" is a mode where an image (static image) of one frame of ultrasound images (video) generated in the past is read from a cine memory 150 described below and displayed.

In the embodiment, a plurality of ultrasound image generation modes are selectable, and specifically, include brightness (B) mode, a color flow (CF) mode, and a pulse wave (PW) mode. The B mode is a mode where amplitude of an ultrasound echo is converted into brightness and a tomographic image is displayed. The CF mode is a mode where an average blood flow speed, flow fluctuation, intensity of a flow signal, flow power, or the like are mapped to various colors and superimposedly displayed on a B mode image. The PW mode is a mode where a speed (for example, a speed of a blood flow) of an ultrasound echo source detected based on transmission and reception of a pulse wave is displayed.

The above-described ultrasound image generation modes are merely examples, and modes other than the above-described three kinds of modes, for example, an amplitude (A) mode, a motion (M) mode, a contrast radiography mode, and the like may be further included.

### <<Configuration of Ultrasound Endoscope 12>>

Next, the configuration of the ultrasound endoscope 12 will be described referring to Fig. 1 described above, and Figs. 2, 3, and 5. Fig. 2 is an enlarged plan view of the distal end portion of the insertion part 22 of the ultrasound endoscope 12 and the periphery of the distal end portion. Fig. 3 is a sectional view showing a cross section of the distal end portion 40 of the insertion part 22 of the ultrasound endoscope 12 taken along the line I-I of Fig. 2.

As described above, the ultrasound endoscope 12 has the insertion part 22 and the operating part 24. As shown in Fig. 1, the insertion part 22 comprises the distal end portion 40, a bending portion 42, and a flexible portion 43 in order from the distal end side (free end side). As shown in Fig. 2, the ultrasound observation part 36 and the endoscope observation part 38 are provided in the distal end portion 40. As shown in Fig. 3, the ultrasound transducer unit 46 comprising a plurality of ultrasound transducers 48 is disposed in the ultrasound observation part 36.

Furthermore, as shown in Fig. 2, a treatment tool lead-out port 44 is provided in the distal end portion 40. The treatment tool lead-out port 44 serves as an outlet of a treatment tool (not shown), such as a forcep, a puncture needle, or a high-frequency scalpel. Furthermore, the treatment tool lead-out port 44 serves as a suction port in sucking aspirates, such as blood or filth inside the body.

The bending portion 42 is a portion consecutively provided on a proximal end side (a side opposite to a side on which the ultrasound transducer unit 46 is provided) than the distal end portion 40, and is freely bent. The flexible portion 43 is a portion that connects the bending portion 42 and the operating part 24, has flexibility, and is provided in an elongated and extended state.

A plurality of pipe lines for air and water supply and a plurality of pipe lines for suction are formed inside each of the insertion part 22 and the operating part 24. In addition, a treatment tool channel 45 of which one end communicates with the treatment tool lead-out port 44 is formed inside each of the insertion part 22 and the operating part 24.

Next, the ultrasound observation part 36, the endoscope observation part 38, the water supply tank 21a, the suction pump 21b, and the operating part 24 among the components of the ultrasound endoscope 12 will be described in detail.

### (Ultrasound Observation part 36)

The ultrasound observation part 36 is a portion that is provided to acquire an ultrasound image, and is disposed on the distal end side in the distal end portion 40 of the insertion part 22. As shown in Fig. 3, the ultrasound observation part 36 comprises the ultrasound transducer unit 46, a plurality of coaxial cables 56, and a flexible printed circuit (FPC) 60.

The ultrasound transducer unit 46 corresponds to an ultrasound probe (probe), transmits ultrasonic waves using an ultrasound transducer array 50, in which a plurality of ultrasound transducers 48 described below are arranged, inside a body cavity of a patient, receives reflected waves (echoes) of the ultrasonic waves reflected by the observation target part, and outputs reception signals. The ultrasound transducer unit 46 according to the embodiment is a convex type, and transmits ultrasonic waves radially (in an arc shape). Note that the type (model) of the ultrasound transducer unit 46 is not particularly limited to the convex type, and other types may be used as long as ultrasonic waves can be transmitted and received. For example, a radial type, a linear type, or the like may be used.

As shown in Fig. 3, the ultrasound transducer unit 46 is configured by laminating a backing material layer 54, the ultrasound transducer array 50, an acoustic matching layer 74, and an acoustic lens 76.

The ultrasound transducer array 50 has a plurality of ultrasound transducers 48 arranged in a one-dimensional array. In more detail, the ultrasound transducer array 50 is configured by arranging N (for example, N = 128) ultrasound transducers 48 at regular intervals in a convex bent shape along an axial direction of the distal end portion 40 (a longitudinal axis direction of the insertion part 22). The ultrasound transducer array 50 may be configured by arranging a plurality of ultrasound transducers 48 in a two-dimensional array.

Each of the N ultrasound transducers 48 is configured by disposing electrodes on both surfaces of a piezoelectric element (piezoelectric body). As the piezoelectric element, barium titanate (BaTiO₃), lead zirconate titanate (PZT), potassium niobate (KNbO₃), or the like is used.

The electrodes have an individual electrode (not shown) individually provided for each of a plurality of ultrasound transducers 48 and a transducer ground (not shown) common to a plurality of ultrasound transducers 48. The electrodes are electrically connected to the ultrasound observation device 14 through the coaxial cables 56 and the FPC 60.

A pulsed drive voltage is supplied as an input signal (transmission signal) from the ultrasound observation device 14 to each ultrasound transducer 48 through the coaxial cables 56. In a case where the drive voltage is applied to the electrodes of the ultrasound transducer 48, the piezoelectric element expands and contracts to drive (vibrate) the ultrasound transducer 48. As a result, a pulsed ultrasonic wave is output from the ultrasound transducer 48. In this case, the amplitude of the ultrasonic wave output from the ultrasound transducer 48 has magnitude according to intensity (output intensity) when the ultrasound transducer 48 outputs the ultrasonic wave. Here, the output intensity is defined as the magnitude of sound pressure of the ultrasonic wave output from the ultrasound transducer 48.

Each ultrasound transducer 48 vibrates (is driven) with reception of a reflected wave (echo) of the ultrasonic wave, and the piezoelectric element of each ultrasound transducer 48 generates an electric signal. The electric signal is output as a reception signal of the ultrasonic wave from the ultrasound transducer 48 toward the ultrasound observation device 14. In this case, the magnitude (voltage value) of the electric signal output from the ultrasound transducer 48 is magnitude according to reception sensitivity when the ultrasound transducer 48 receives the ultrasonic wave. Here, the reception sensitivity is defined as a ratio of the amplitude of the electric signal output from the ultrasound transducer 48 with reception of the ultrasonic wave to the amplitude of the ultrasonic wave transmitted from the ultrasound transducer 48.

In the embodiment, the N ultrasound transducers 48 are driven sequentially by an electronic switch, such as a multiplexer 140 (see Fig. 5), scanning with ultrasonic waves is performed in a scanning range along a curved surface on which the ultrasound transducer array 50 is disposed, for example, a range of about several tens of mm from the center of curvature of the curved surface. In more detail, in a case where a B mode image (tomographic image) is acquired as an ultrasound image, the drive voltage is supplied to m (for example, m = N/2) continuously arranged ultrasound transducers 48 (hereinafter, referred to as drive target transducers) among the N ultrasound transducers 48 by selection of opening channel selection of the multiplexer 140. With this, the m drive target transducers are driven, and an ultrasonic wave is output from each drive target transducer of the opening channel. The ultrasonic waves output from the m drive target transducers are immediately composed, and the composite wave (ultrasound beam) is transmitted toward the observation target part. Thereafter, the m drive target transducers receive ultrasonic waves (echoes) reflected by the observation target part and output electric signals (reception signals) according to reception sensitivity at that moment.

Then, the above-described series of steps (that is, the supply of the drive voltage, the transmission and reception of the ultrasonic waves, and the output of the electric signal) are repeatedly performed while shifting the positions of the drive target transducers among the N ultrasound transducers 48 one by one (one ultrasound transducer 48 at a time). Specifically, the above-described series of steps are started from m drive target transducers on both sides of the ultrasound transducer 48 positioned at one end among the N ultrasound transducers 48. Then, the above-described series of steps are repeated each time the positions of the drive target transducers are shifted due to switching of the opening channel by the multiplexer 140. Finally, the above-described series of steps are repeatedly performed N times in total up to m drive target transducers on both sides of the ultrasound transducer 48 positioned at the other end among the N ultrasound transducers 48.

The backing material layer 54 supports each ultrasound transducer 48 of the ultrasound transducer array 50 from a rear surface side. Furthermore, the backing material layer 54 has a function of attenuating ultrasonic waves propagating to the backing material layer 54 side among ultrasonic waves emitted from the ultrasound transducers 48 or ultrasonic waves (echoes) reflected by the observation target part. A backing material is a material having rigidity, such as hard rubber, and an ultrasonic wave attenuation material (ferrite, ceramics, or the like) is added as necessary.

The acoustic matching layer 74 is superimposed on the ultrasound transducer array 50, and is provided for acoustic impedance matching between the body of the patient and the ultrasound transducer 48. The acoustic matching layer 74 is provided, whereby it is possible to increase the transmittance of the ultrasonic wave. As a material of the acoustic matching layer 74, various organic materials of which a value of acoustic impedance is closer to that of the body of the patient than the piezoelectric element of the ultrasound transducer 48 can be used. As the material of the acoustic matching layer 74, specifically, epoxy-based resin, silicone rubber, polyimide, polyethylene, and the like are exemplified.

The acoustic lens 76 superimposed on the acoustic matching layer 74 converges ultrasonic waves emitted from the ultrasound transducer array 50 toward the observation target part. The acoustic lens 76 is made of, for example, silicone-based resin (millable silicone rubber (HTV rubber), liquid silicone rubber (RTV rubber), or the like), butadiene-based resin, polyurethane-based resin, or the like, and powder of titanium oxide, alumina, silica, or the like is mixed as necessary.

The FPC 60 is electrically connected to the electrodes of each ultrasound transducer 48. Each of a plurality of coaxial cables 56 is wired to the FPC 60 at one end. Then, in a case where the ultrasound endoscope 12 is connected to the ultrasound observation device 14 through the ultrasound connector 32a, each of a plurality of coaxial cables 56 is electrically connected to the ultrasound observation device 14 at the other end (a side opposite to the FPC 60 side).

### (Endoscope Observation part 38)

The endoscope observation part 38 is a portion that is provided to acquire an endoscope image, and is disposed on a proximal end side than the ultrasound observation part 36 in the distal end portion 40 of the insertion part 22. As shown in Figs. 2 and 3, the endoscope observation part 38 is configured with the observation window 82, the objective lens 84, the solid-state imaging element 86, the illumination windows 88, a cleaning nozzle 90, a wiring cable 92, and the like.

The observation window 82 is attached in a state inclined with respect to the axial direction (the longitudinal axis direction of the insertion part 22) in the distal end portion 40 of the insertion part 22. Light reflected by the observation target adjacent part and incident from the observation window 82 is formed on an imaging surface of the solid-state imaging element 86 by the objective lens 84.

The solid-state imaging element 86 photoelectrically converts reflected light of the observation target adjacent part transmitted through the observation window 82 and the objective lens 84 and formed on the imaging surface, and outputs an imaging signal. As the solid-state imaging element 86, a charge coupled device (CCD), a complementary metal oxide semiconductor (CMOS), or the like can be used. A captured image signal output from the solid-state imaging element 86 is transmitted to the endoscope processor 16 by the universal cord 26 by way of the wiring cable 92 extending from the insertion part 22 to the operating part 24.

The illumination windows 88 are provided at both side positions of the observation window 82. An exit end of the light guide (not shown) is connected to the illumination windows 88. The light guide extends from the insertion part 22 to the operating part 24, and an incidence end of the light guide is connected to the light source device 18 connected through the universal cord 26. Illumination light emitted from the light source device 18 is transmitted through the light guide, and the observation target adjacent part is irradiated with illumination light from the illumination windows 88.

The cleaning nozzle 90 is an ejection hole formed in the distal end portion 40 of the insertion part 22 to clean the surfaces of the observation window 82 and the illumination windows 88, and air or a cleaning liquid is ejected from the cleaning nozzle 90 toward the observation window 82 and the illumination windows 88. In the embodiment, the cleaning liquid ejected from the cleaning nozzle 90 is water, in particular, degassed water. Note that the cleaning liquid is not particularly limited, and other liquids, for example, normal water (water that is not degassed) may be used.

### (Water Supply Tank 21a and Suction Pump 21b)

The water supply tank 21a is a tank that stores degassed water, and is connected to the light source connector 32c by an air and water supply tube 34a. Degassed water is used as the cleaning liquid that is ejected from the cleaning nozzle 90.

The suction pump 21b sucks aspirates (including degassed water supplied for cleaning) into the body cavity through the treatment tool lead-out port 44. The suction pump 21b is connected to the light source connector 32c by a suction tube 34b. The ultrasound endoscope system 10 may comprise an air supply pump that supplies air to a predetermined air supply destination, or the like.

Inside the insertion part 22 and the operating part 24, the treatment tool channel 45 and an air and water supply pipe line (not shown) are provided.

The treatment tool channel 45 communicates a treatment tool insertion port 30 and the treatment tool lead-out port 44 provided in the operating part 24. Furthermore, the treatment tool channel 45 is connected to a suction button 28b provided in the operating part 24. The suction button 28b is connected to the suction pump 21b in addition to the treatment tool channel 45.

The air and water supply pipe line communicates with the cleaning nozzle 90 on one end side, and is connected to an air and water supply button 28a provided in the operating part 24 on the other end side. The air and water supply button 28a is connected to the water supply tank 21a in addition to the air and water supply pipe line.

### (Operating Part 24)

The operating part 24 is a portion that is operated by the operator at the time of a start of ultrasonography, during diagnosis, at the time of an end of diagnosis, and the like, and has one end to which one end of the universal cord 26 is connected. Furthermore, as shown in Fig. 1, the operating part 24 has the air and water supply button 28a, the suction button 28b, a pair of angle knobs 29, and a treatment tool insertion port (forceps port) 30.

In a case where each of a pair of angle knobs 29 is moved rotationally, the bending portion 42 is remotely operated to be bent and deformed. With the deformation operation, it is possible to direct the distal end portion 40 of the insertion part 22, in which the ultrasound observation part 36 and the endoscope observation part 38 are provided, to a desired direction.

The treatment tool insertion port 30 is a hole formed such that the treatment tool (not shown), such as a forcep, is inserted thereinto, and communicates with the treatment tool lead-out port 44 through the treatment tool channel 45. The treatment tool inserted into the treatment tool insertion port 30 is introduced from the treatment tool lead-out port 44 into the body cavity after passing through the treatment tool channel 45.

The air and water supply button 28a and the suction button 28b are two-stage switching type push buttons, and are operated to switch opening and closing of the pipe line provided inside each of the insertion part 22 and the operating part 24.

### <<Schematic Configuration of Endoscope Processor 16»

Here, although description of the detailed configuration of the endoscope processor 16 will not be repeated, the endoscope processor 16 comprises an endoscope image recognition unit 170 in addition to general components known in the related art for capturing an endoscope image.

The endoscope image recognition unit 170 learns a relationship between an endoscope image for learning and a lesion region displayed in the endoscope image for learning, on a plurality of endoscope images for learning in advance and recognizes the lesion region displayed in the endoscope image for diagnosis from the endoscope image for diagnosis generated by the endoscope processor 16 based on a learning result.

The endoscope image for learning is an existing endoscope image that is used for the endoscope image recognition unit 170 to learn a relationship between an endoscope image and a lesion region displayed in the endoscope image, and for example, various endoscope images captured in the past can be used.

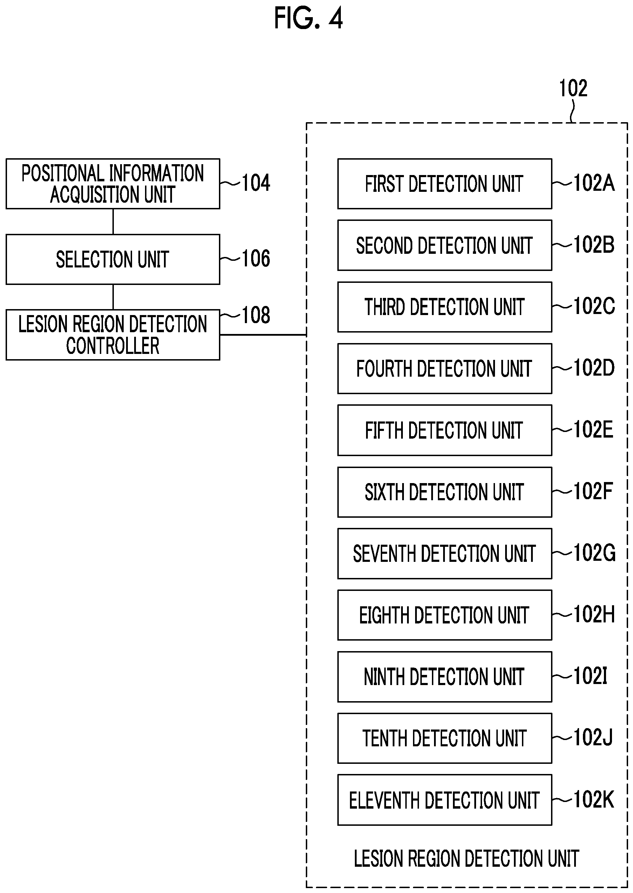

As shown in Fig. 4, the endoscope image recognition unit 170 comprises a lesion region detection unit 102, a positional information acquisition unit 104, a selection unit 106, and a lesion region detection controller 108.

The lesion region detection unit 102 detects the lesion region from the endoscope image for diagnosis based on the learning result. The lesion region detection unit 102 comprises a plurality of detection units corresponding to a plurality of positions in the body cavity. Here, as an example, as shown in Fig. 4, the lesion region detection unit 102 comprises first to eleventh detection units 102A to 102K. The first detection unit 102A corresponds to rectum, the second detection unit 102B corresponds to an S-shaped colon, the third detection unit 102C corresponds to a descending colon, the fourth detection unit 102D corresponds to a transverse colon, the fifth detection unit 102E corresponds to an ascending colon, the sixth detection unit 102F corresponds to a cecum, the seventh detection unit 102G corresponds to ileum, the eighth detection unit 102H corresponds to a jejunum, the ninth detection unit 1021 corresponds to a duodenum, the tenth detection unit 102J corresponds to a stomach, and the eleventh detection unit 102K corresponds to an esophagus.

The first to eleventh detection units 102A to 102K are learned models. A plurality of learned models are models learned using respective data sets having different endoscope images for learning. In detail, a plurality of learned models are models learned a relationship between an endoscope image for learning and a lesion region displayed in the endoscope image for learning in advance using respective data sets having endoscope images for learning obtained by imaging different positions in the body cavity.

That is, the first detection unit 102A is a model learned using a data set having endoscope images for learning of rectum, the second detection unit 102B is a model that learns using a data set having endoscope images for learning of an S-shaped colon, the third detection unit 102C is a model that learns using a data set having endoscope images for learning of a descending colon, the fourth detection unit 102D is a model that learns using a data set having endoscope images for learning of a transverse colon, the fifth detection unit 102E is a model that learns using a data set having endoscope images for learning of an ascending colon, the sixth detection unit 102F is a model that learns using a data set having endoscope images for learning of a cecum, the seventh detection unit 102G is a model that learns using a data set having endoscope images for learning of ileum, the eighth detection unit 102H is a model that learns using a data set having endoscope images for learning of a jejunum, the ninth detection unit 1021 is a model that learns using a data set having endoscope images for learning of a duodenum, the tenth detection unit 102J is a model that learns using a data set having endoscope images for learning of a stomach, and the eleventh detection unit 102K is a model that learns using a data set having endoscope images for learning of an esophagus.

A learning method is not particularly limited as long as it is possible to learn the relationship between the endoscope image and the lesion region from a plurality of endoscope images for learning, and to generate a learned model.

As the learning method, for example, deep learning that uses a hierarchical structure type neural network as an example of machine learning, which is one of artificial intelligence (AI) techniques, can be used.

Machine learning other than deep learning may be used, an artificial intelligence technique other than machine learning may be used, or a learning method other than an artificial intelligence technique may be used.

A learned model may be generated using only the endoscope images for learning. In this case, the learned model is not updated, and the same learned model can be used constantly.

Alternatively, a configuration may be made in which a learned model is generated using endoscope images for diagnosis in addition to the endoscope images for learning. In this case, a learned model is updated at any time by learning a relationship between an endoscope image for diagnosis and a lesion region displayed in the endoscope image for diagnosis.

Subsequently, the positional information acquisition unit 104 acquires information regarding a position in a body cavity of an endoscope image. Here, the operator, such as a physician, input information regarding the position using the console 100. The positional information acquisition unit 104 acquires information regarding the position input from the console 100.

As information regarding a position in a body cavity of an image, information, such as rectum, an S-shaped colon, a descending colon, a transverse colon, an ascending colon, a cecum, ileum, a jejunum, a duodenum, a stomach, and an esophagus, is input. A configuration may be made in which such position candidates are selectably displayed on the monitor 20, and the operator, such as a physician, selects the position using the console 100.

Subsequently, the selection unit 106 selects a detection unit corresponding to information regarding the position acquired by the positional information acquisition unit 104, from the lesion region detection unit 102. That is, the selection unit 106 selects the first detection unit 102A in a case where information regarding the position is rectum, selects the second detection unit 102B in a case where information regarding the position is an S-shaped colon, selects the third detection unit 102C in a case where information regarding the position is a descending colon, selects the fourth detection unit 102D in a case where information regarding the position is a transverse colon, selects the fifth detection unit 102E in a case where information regarding the position is an ascending colon, selects the sixth detection unit 102F in a case where information regarding the position is a cecum, selects the seventh detection unit 102G in a case where information regarding the position is ileum, selects the eighth detection unit 102H in a case where information regarding the position is a jejunum, selects the ninth detection unit 1021 in a case where information regarding the position is a duodenum, selects the tenth detection unit 102J in a case where information regarding the position is a stomach, and selects the eleventh detection unit 102K in a case where information regarding the position is an esophagus.

Subsequently, the lesion region detection controller 108 makes the detection unit selected by the selection unit 106 detect a lesion region from the endoscope image. The lesion region herein is not limited to a region caused by illness, and includes a region in a state different from a normal state in appearance. Examples of the lesion region include a polyp, cancer, a colon diverticulum, inflammation, a scar from treatment, such as endoscopic mucosal resection (EMR) scar or endoscopic submucosal dissection (ESD) scar, a clipped spot, a bleeding point, perforation, and angiodysplasia.

In the endoscope image recognition unit 170, the positional information acquisition unit 104 acquires information regarding the position in the body cavity of the endoscope image.

Subsequently, the selection unit 106 selects a detection unit corresponding to information regarding the position acquired by the positional information acquisition unit 104, from the lesion region detection unit 102.

Subsequently, the lesion region detection controller 108 performs control such that the detection unit selected by the selection unit 106 detects the lesion region from the endoscope image for diagnosis based on a learning result.

### «Configuration of Ultrasound Observation Device 14»

The ultrasound observation device 14 makes the ultrasound transducer unit 46 transmit and receive ultrasonic waves and generates an ultrasound image by imaging reception signals output from the ultrasound transducers 48 (in detail, the drive target elements) at the time of reception of the ultrasonic waves. The ultrasound observation device 14 displays the endoscope image transferred from the endoscope processor 16, the anatomical schema diagram, and the like on the monitor 20, in addition to the generated ultrasound image.

As shown in Fig. 5, the ultrasound observation device 14 has the multiplexer 140, the reception circuit 142, the transmission circuit 144, an A/D converter 146, an application specific integrated circuit (ASIC) 148, the cine memory 150, a central processing unit (CPU) 152, a digital scan converter (DSC) 154, an ultrasound image recognition unit 168, and the display controller 172.

The reception circuit 142 and the transmission circuit 144 are electrically connected to the ultrasound transducer array 50 of the ultrasound endoscope 12. The multiplexer 140 selects a maximum of m drive target transducers from among the N ultrasound transducers 48 and opens the channels.

The transmission circuit 144 has a field programmable gate array (FPGA), a pulser (pulse generation circuit 158), a switch (SW), and the like, and is connected to the multiplexer 140 (MUX). An application-specific integrated circuit (ASIC), instead of the FPGA, may be used.

The transmission circuit 144 is a circuit that supplies a drive voltage for ultrasonic wave transmission to the drive target transducers selected by the multiplexer 140 in response to a control signal sent from the CPU 152 for transmission of ultrasonic waves from the ultrasound transducer unit 46. The drive voltage is a pulsed voltage signal (transmission signal), and is applied to the electrodes of the drive target transducers through the universal cord 26 and the coaxial cables 56.

The transmission circuit 144 has a pulse generation circuit 158 that generates a transmission signal based on a control signal. Under the control of the CPU 152, the transmission circuit 144 generates a transmission signal for driving a plurality of ultrasound transducers 48 to generate ultrasonic waves using the pulse generation circuit 158 and supplies the transmission signal to a plurality of ultrasound transducers 48. In more detail, under the control of the CPU 152, in a case of performing ultrasonography, the transmission circuit 144 generates a transmission signal having a drive voltage for performing ultrasonography using the pulse generation circuit 158.

The reception circuit 142 is a circuit that receives electric signals output from the drive target transducers, which receive the ultrasonic waves (echoes), that is, reception signals. Furthermore, the reception circuit 142 amplifies reception signals received from the ultrasound transducers 48 in response to a control signal sent from the CPU 152 and delivers the signals after amplification to the A/D converter 146. The A/D converter 146 is connected to the reception circuit 142, converts the reception signals received from the reception circuit 142 from analog signals to digital signals and outputs the digital signals after conversion to the ASIC 148.

The ASIC 148 is connected to the A/D converter 146. As shown in Fig. 5, the ASIC 148 configures a phase matching unit 160, a B mode image generation unit 162, a PW mode image generation unit 164, a CF mode image generation unit 166, and a memory controller 151.

In the embodiment, although the above-described functions (specifically, the phase matching unit 160, the B mode image generation unit 162, the PW mode image generation unit 164, the CF mode image generation unit 166, and the memory controller 151) are realized by a hardware circuit, such as the ASIC 148, the invention is not limited thereto. The above-described functions may be realized by making a central processing unit (CPU) and software (computer program) for executing various kinds of data processing cooperate with each other.

The phase matching unit 160 executes processing of giving a delay time to the reception signals (reception data) digitized by the A/D converter 146 and performing phasing addition (performing addition after matching the phases of the reception data). With the phasing addition processing, sound ray signals in which the focus of the ultrasound echo is narrowed are generated.

The B mode image generation unit 162, the PW mode image generation unit 164, and the CF mode image generation unit 166 generate an ultrasound image based on the electric signals (strictly, sound ray signals generated by phasing addition on the reception data) output from the drive target transducers among a plurality of ultrasound transducers 48 when the ultrasound transducer unit 46 receives the ultrasonic waves.

The B mode image generation unit 162 is an image generation unit that generates a B mode image as a tomographic image of the inside (the inside of the body cavity) of the patient. The B mode image generation unit 162 performs correction of attenuation due to a propagation distance on each of the sequentially generated sound ray signals according to a depth of a reflection position of the ultrasonic wave through sensitivity time gain control (STC). Furthermore, the B mode image generation unit 162 executes envelope detection processing and logarithm (Log) compression processing on the sound ray signal after correction to generate a B mode image (image signal).

The PW mode image generation unit 164 is an image generation unit that generates an image indicating a speed of a blood flow in a predetermined direction. The PW mode image generation unit 164 extracts a frequency component by performs fast Fourier transform on a plurality of sound ray signals in the same direction among the sound ray signals sequentially generated by the phase matching unit 160. Thereafter, the PW mode image generation unit 164 calculates the speed of the blood flow from the extracted frequency component and generates a PW mode image (image signal) indicating the calculated speed of the blood flow.

The CF mode image generation unit 166 is an image generation unit that generates an image indicating information regarding a blood flow in a predetermined direction. The CF mode image generation unit 166 generates an image signal indicating information regarding the blood flow by obtaining autocorrelation of a plurality of sound ray signals in the same direction among the sound ray signals sequentially generated by the phase matching unit 160. Thereafter, the CF mode image generation unit 166 generates a CF mode image (image signal) as a color image, in which information relating to the blood flow is superimposed on the B mode image generated by the B mode image generation unit 162, based on the above-described image signal.

The memory controller 151 stores the image signal generated by the B mode image generation unit 162, the PW mode image generation unit 164, or the CF mode image generation unit 166 in the cine memory 150.

The DSC 154 is connected to the ASIC 148, converts (raster conversion) the signal of the image generated by the B mode image generation unit 162, the PW mode image generation unit 164, or the CF mode image generation unit 166 into an image signal compliant with a normal television signal scanning system, executes various kinds of necessary image processing, such as gradation processing, on the image signal, and then, outputs the image signal to the ultrasound image recognition unit 168.

The ultrasound image recognition unit 168 learns at least one of a relationship between an ultrasound image for learning and a name of an organ (a name of an observation target part) displayed in the ultrasound image for learning or a relationship between the ultrasound image for learning and a position and an orientation of the distal end portion 40 of the ultrasound endoscope at the time of imaging of the ultrasound image for learning, on a plurality of ultrasound images for learning in advance, and recognizes at least one of a name of an organ displayed in an ultrasound image for diagnosis or the position and the orientation of the distal end portion 40 of the ultrasound endoscope 12 from the ultrasound image for diagnosis generated by the ultrasound observation device 14 based on the learning result.

The ultrasound image for learning is an existing ultrasound image that is used for the ultrasound image recognition unit 168 to learn the relationship of the ultrasound image, the name of the organ, and the position and the orientation of the distal end portion 40 of the ultrasound endoscope 12, and for example, various ultrasound images captured in the past can be used.

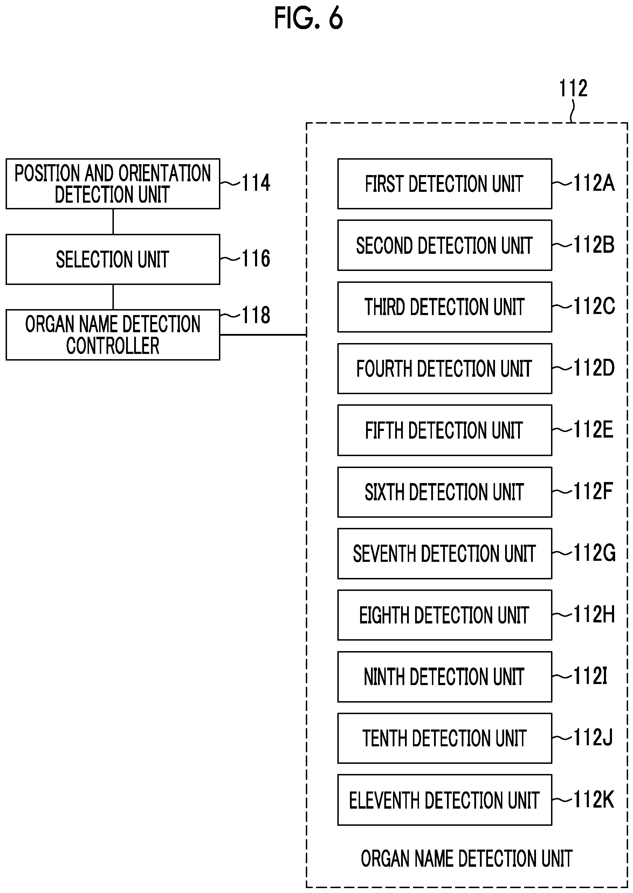

As shown in Fig. 6, the ultrasound image recognition unit 168 comprises an organ name detection unit 112, a position and orientation detection unit 114, a selection unit 116, and an organ name detection controller 118.

The organ name detection unit 112 detects the name of the organ displayed in the ultrasound image for diagnosis from the ultrasound image for diagnosis based on a learning result. The organ name detection unit 112 comprises a plurality of detection units corresponding to a plurality of positions to be an observation target part in the body of the subject. Here, as an example, the organ name detection unit 112 comprises first to eleventh detection units 112A to 112K. The first detection unit 112A corresponds to a confluence of an aorta, a celiac artery, and a superior mesenteric artery, the second detection unit 112B corresponds to a pancreatic body, the third detection unit 112C corresponds to a pancreatic tail, the fourth detection unit 112D corresponds to a confluence of a splenic vein, a superior mesenteric vein, and a portal vein, the fifth detection unit 112E corresponds to a pancreatic head, the sixth detection unit 112F corresponds to a gallbladder, the seventh detection unit 112G corresponds to a portal vein, the eighth detection unit 112H corresponds to a common bile duct, the ninth detection unit 1121 corresponds to a gallbladder, the tenth detection unit 112J corresponds to a pancreatic uncinate process, and the eleventh detection unit 112K corresponds to a papilla.

The first to eleventh detection units 112A to 112K are learned models. A plurality of learned models are models learned using respective data sets having different ultrasound images for learning. In detail, a plurality of learned models are models learned a relationship between an ultrasound image for learning and a name of an organ displayed in the ultrasound image for learning in advance using data sets having ultrasound images for learning obtained by imaging different positions each to be an observation target part in the body of the subject.

That is, the first detection unit 112A is a model learned using a data set having ultrasound images for learning of a confluence of an aorta, a celiac artery, and a superior mesenteric artery, the second detection unit 112B is a model learned using a data set having ultrasound images for learning of a pancreatic body, the third detection unit 112C is a model learned using a data set having ultrasound images for learning of a pancreatic tail, the fourth detection unit 112D is a model learned using a data set having ultrasound images for learning of a confluence of a splenic vein, a superior mesenteric vein, and a portal vein, the fifth detection unit 112E is a model learned using a data set having ultrasound images for learning of a pancreatic head, the sixth detection unit 112F is a model learned using a data set having ultrasound images for learning of a gallbladder, the seventh detection unit 112G is a model learned using a data set having ultrasound images for learning of a portal vein, the eighth detection unit 112H is a model learned using a data set having ultrasound images for learning of a common bile duct, the ninth detection unit 1121 is a model learned using a data set having ultrasound images for learning of a gallbladder, the tenth detection unit 112J is a model learned using a data set having ultrasound images for learning of a pancreatic uncinate process, and the eleventh detection unit 112K is a model learned using a data set having ultrasound images for learning of a papilla.

An observation route (a movement route of the distal end portion 40 of the ultrasound endoscope 12) in the body in a case of capturing an ultrasound image and representative observation points (the position and the orientation of the distal end portion 40 of the ultrasound endoscope 12) are generally determined. For this reason, it is possible to learn an ultrasound image at a representative observation point, a name of an organ displayed in the ultrasound image, and a position and an orientation of the distal end portion 40 of the ultrasound endoscope 12 at the observation point in association with one another.

Hereinafter, a representative observation point (the position and the orientation of the distal end portion 40 of the ultrasound endoscope 12) in the body in a case of capturing an ultrasound image will be described.

Examples of the representative observation point in the body include (1) to (11) described below.
(1) confluence of aorta, celiac artery, and superior mesenteric artery
(2) pancreatic body
(3) pancreatic tail
(4) confluence of splenic vein, superior mesenteric vein, and portal vein
(5) pancreatic head
(6) gallbladder
(7) portal vein
(8) common bile duct
(9) gallbladder
(10) pancreatic uncinate process
(11) papilla

Here, (1) confluence of aorta, celiac artery, and superior mesenteric artery, (2) pancreatic body, (3) pancreatic tail, (4) confluence of splenic vein, superior mesenteric vein, and portal vein, (5) pancreatic head, and (6) gallbladder are representative observation points from a stomach, (7) portal vein, (8) common bile duct, and (9) gallbladder are representative observation points from a duodenal bulb, and (10) pancreatic uncinate process and (11) papilla are representative observation points from a pars descendens duodeni.

For example, an observation procedure in a case of performing observation in an order of (1) confluence of aorta, celiac artery, and superior mesenteric artery, (3) pancreatic tail, (4) confluence of splenic vein, superior mesenteric vein, and portal vein, and (5) pancreatic head as the observation points will be described.

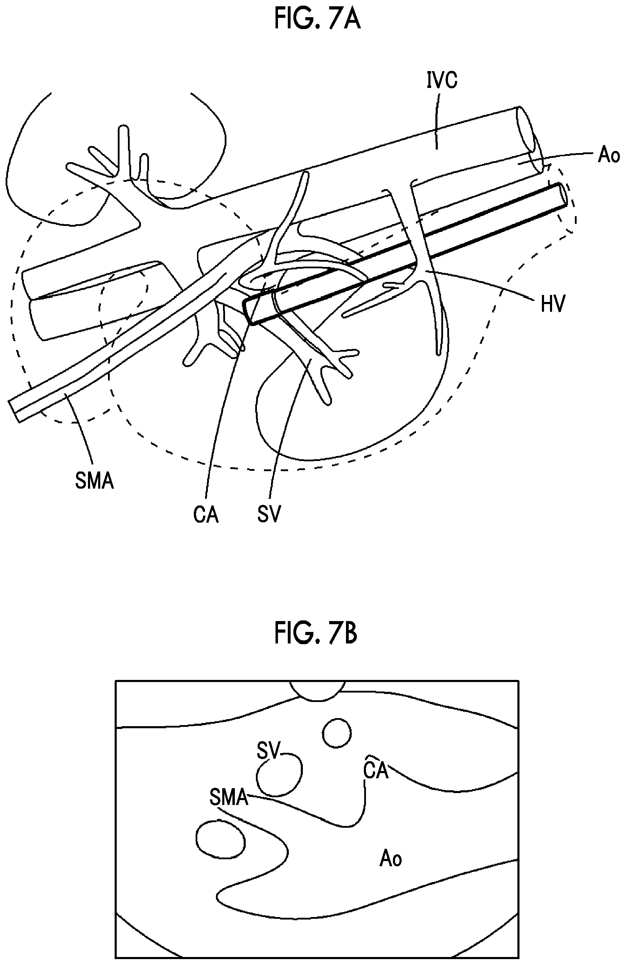

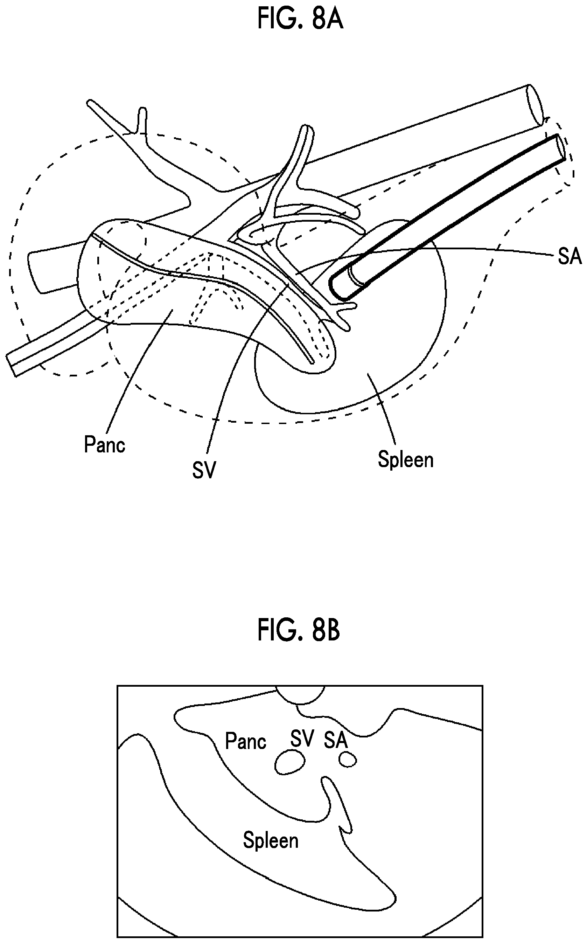

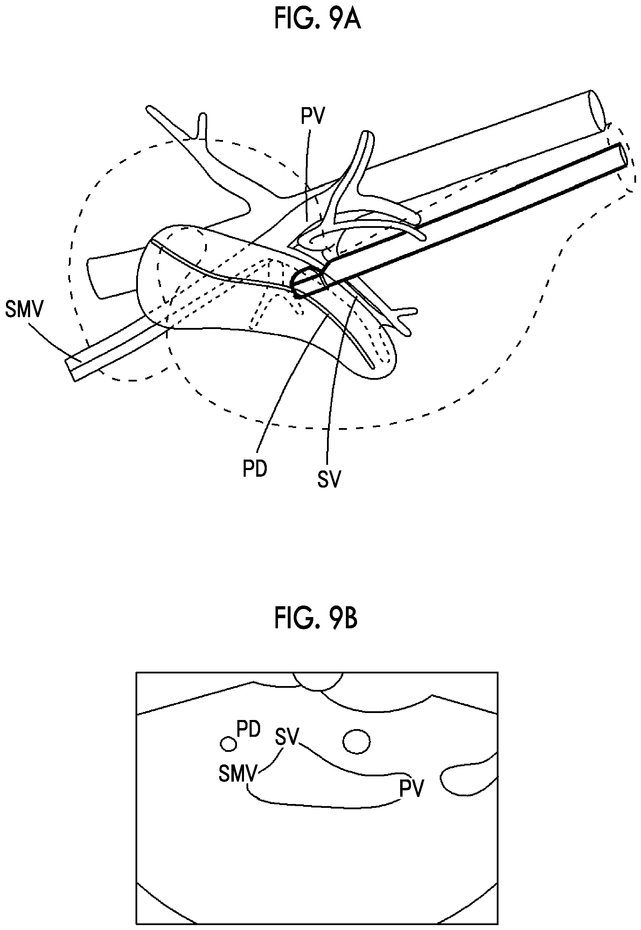

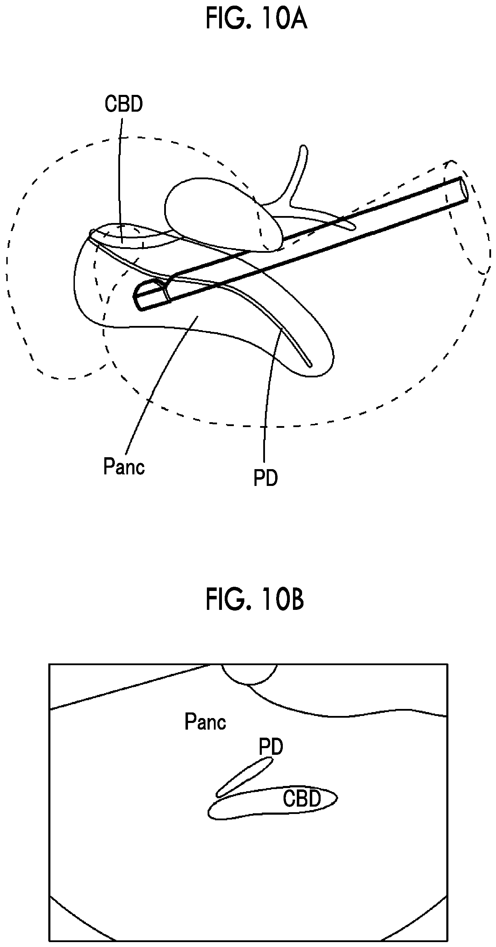

Fig. 7A is an anatomical schema diagram of an embodiment representing a confluence of an aorta, a celiac artery, and a superior mesenteric artery, and Fig. 7B is a conceptual diagram of an embodiment representing an ultrasound image of the confluence of the aorta, the celiac artery, and the superior mesenteric artery. Fig. 8A is an anatomical schema diagram of an embodiment representing a pancreatic tail, and Fig. 8B is a conceptual diagram of an embodiment representing an ultrasound image of the pancreatic tail. Fig. 9A is an anatomical schema diagram of an embodiment representing a confluence of a splenic vein, a superior mesenteric vein, and a portal vein, and Fig. 9B is a conceptual diagram of an embodiment representing an ultrasound image of the confluence of the splenic vein, the superior mesenteric vein, and the portal vein. Fig. 10A is an anatomical schema diagram of an embodiment representing a pancreatic head, and Fig. 10B is a conceptual diagram of an embodiment representing an ultrasound image of the pancreatic head.

In Figs. 7A and 7B, HV represents a hepatic vein, IVC represents an inferior vena cava, Ao represents an aorta, CA represents a celiac artery, SMA represents a superior mesenteric artery, and SV represents a splenic vein.

In a case of observing (1) confluence of aorta, celiac artery, and superior mesenteric artery, in a case where the distal end portion 40 of the insertion part 22 of the ultrasound endoscope 12 is rotated clockwise while following the hepatic vein, the inferior vena cava is visualized. In a case where the distal end portion 40 is further rotated clockwise, the aorta is visualized. In a case where the distal end portion 40 is further pushed forward along the aorta, as shown in Figs. 7A and 7B, the celiac artery and the superior mesenteric artery are visualized.

In Figs. 8A and 8B, SA represents a splenic artery, SV represents a splenic vein, Panc represents pancreas, and Spleen represents a spleen.

Subsequently, in a case of observing (3) pancreatic tail, in a case where the distal end portion 40 is further rotated clockwise, the pancreatic body, the splenic artery, and the splenic vein are visualized. In a case where the distal end portion 40 is further rotated clockwise, the pancreatic body/tail and a left kidney are visualized. In a case where the distal end portion 40 is rotated clockwise while following the splenic artery and the splenic vein, and is up-angled, as shown in Figs. 8A and 8B, the pancreatic tail to a splenic hilum are visualized.

In Figs. 9A and 9B, SV represents a splenic vein, SMV represents a superior mesenteric vein, PV represents a portal vein, and PD represents a pancreatic duct.

Subsequently, in a case of observing (4) confluence of splenic vein, superior mesenteric vein, and portal vein, in a case where the distal end portion 40 is rotated counterclockwise in an up-angled state, a left adrenal gland is visualized. In a case where the distal end portion 40 is rotated counterclockwise from the pancreatic tail while following the splenic vein, the pancreatic body is visualized. In a case where the distal end portion 40 further follows the splenic vein, as shown in Figs. 9A and 9B, the confluence of the splenic vein, the superior mesenteric vein, and the portal vein is visualized.

In Figs. 10A and 10B, PD represents a pancreatic duct, CBD represents a common bile duct, and Panc represents pancreas.

Subsequently, in a case of observing (5) pancreatic head, in a case where the distal end portion 40 is pushed forward while rotating counterclockwise, and follows the pancreatic duct from the confluence of the splenic vein, the superior mesenteric vein, and the portal vein, as shown in Figs. 10A and 10B, a pancreatic head-body transitional area, a main pancreatic duct, and the common bile duct are visualized.

Although an example of observation points in a case of performing observation has been described, the invention is not limited thereto, and the operator can observe desired observation points in a desired order.

Subsequently, the position and orientation detection unit 114 detects the position and the orientation of the distal end portion 40 of the ultrasound endoscope 12 from the ultrasound image for diagnosis based on the learning result.

As the position of the distal end portion 40 of the ultrasound endoscope 12, for example, the above-described observation points, that is, (1) confluence of aorta, celiac artery, and superior mesenteric artery, (2) pancreatic body, (3) pancreatic tail, (4) confluence of splenic vein, superior mesenteric vein, and portal vein, (5) pancreatic head, and (6) gallbladder (representative observation points from the stomach), (7) portal vein, (8) common bile duct, and (9) gallbladder (representative observation points of the duodenal bulb), and (10) pancreatic uncinate process and (11) papilla (representative observation points of the pars descendens duodeni) are detected.

As the orientation of the distal end portion 40 of the ultrasound endoscope 12, the orientations of the distal end portion 40 of the ultrasound endoscope 12 in a case of observing the parts (1) to (11) described above are detected.

The position and orientation detection unit 114 is a learned model. The learned model is a model learned a relationship of an ultrasound image for learning and the position and the orientation of the distal end portion 40 of the ultrasound endoscope at the time of imaging of the ultrasound image for learning, on a plurality of ultrasound images for learning in advance using a data set having endoscope images for learning obtained by imaging different positions each to be an observation target part in the body of the subject.

A learning method is not particularly limited as long as it is possible to learn the relationship of the ultrasound image, the name of the organ, and the position and the orientation of the distal end portion 40 of the ultrasound endoscope 12 from a plurality of ultrasound images for learning, and to generate a learned model.

As the learning method, for example, deep learning that uses a hierarchical structure type neural network as an example of machine learning, which is one of artificial intelligence (AI) techniques, can be used.

Machine learning other than deep learning may be used, an artificial intelligence technique other than machine learning may be used, or a learning method other than an artificial intelligence technique may be used.

A learned model may be generated using only the ultrasound images for learning. In this case, the learned model is not updated, and the same learned model can be used constantly.

Alternatively, a configuration may be made in which a learned model is generated using the ultrasound images for diagnosis in addition to the ultrasound images for learning. In this case, a learned model is updated at any time by learning a relationship of an ultrasound image for diagnosis, a name of an organ displayed in the ultrasound image for diagnosis, and a position and an orientation of the distal end portion 40 of the ultrasound endoscope 12 when the ultrasound image for diagnosis is captured.

It is not essential that the ultrasound image recognition unit 168, and in addition, the position and orientation detection unit 114 detects the orientation of the distal end portion 40 of the ultrasound endoscope 12.

As in the embodiment, in a case where the ultrasound transducer unit 46 is a convex type, a transmission direction of an ultrasonic wave changes with the orientation of the distal end portion 40 of the ultrasound endoscope 12, and thus, it is desirable to detect the orientation of the distal end portion 40 of the ultrasound endoscope 12.

On the other hand, in a case where the ultrasound transducer unit 46 is a radial type, an ultrasonic wave is transmitted over the entire circumference in a radial direction of the ultrasound endoscope 12 regardless of the orientation of the distal end portion 40 of the ultrasound endoscope 12, and thus, there is no need to detect the orientation of the distal end portion 40 of the ultrasound endoscope 12.

Subsequently, the selection unit 116 selects a detection unit corresponding to the position of the distal end portion 40 of the ultrasound endoscope 12 detected by the position and orientation detection unit 114, from the organ name detection unit 112.

That is, the selection unit 116 selects the first detection unit 112A in a case where the position of the distal end portion 40 of the ultrasound endoscope 12 is (1) confluence of aorta, celiac artery, and superior mesenteric artery, selects the second detection unit 112B in a case where the position of the distal end portion 40 of the ultrasound endoscope 12 is (2) pancreatic body, selects the third detection unit 112C in a case where the position of the distal end portion 40 of the ultrasound endoscope 12 is (3) pancreatic tail, selects the fourth detection unit 112D in a case where the position of the distal end portion 40 of the ultrasound endoscope 12 is (4) confluence of splenic vein, superior mesenteric vein, and portal vein, selects the fifth detection unit 112E in a case where the position of the distal end portion 40 of the ultrasound endoscope 12 is (5) pancreatic head, selects the sixth detection unit 112F in a case where the position of the distal end portion 40 of the ultrasound endoscope 12 is (6) gallbladder, selects the seventh detection unit 112G in a case where the position of the distal end portion 40 of the ultrasound endoscope 12 is (7) portal vein, selects the eighth detection unit 112H in a case where the position of the distal end portion 40 of the ultrasound endoscope 12 is (8) common bile duct, selects the ninth detection unit 1121 in a case where the position of the distal end portion 40 of the ultrasound endoscope 12 is (9) gallbladder, selects the tenth detection unit 112J in a case where the position of the distal end portion 40 of the ultrasound endoscope 12 is (10) pancreatic uncinate process, and selects the eleventh detection unit 112K in a case where the position of the distal end portion 40 of the ultrasound endoscope 12 is (11) papilla.

Subsequently, the organ name detection controller 118 makes the detection unit selected by the selection unit 116 detect a name of an organ displayed in the ultrasound image for diagnosis from the ultrasound image for diagnosis. As the name of the organ, while all observation target parts in the body of the subject that can be observed using the ultrasound observation device 14 are included, for example, a liver, pancreas, a spleen, a kidney, an adrenal gland, an aorta, a celiac artery, a splenic artery, a superior mesenteric artery, an inferior vena cava, a hepatic vein, a portal vein, a splenic vein, a superior mesenteric vein, a gallbladder, a common bile duct, a pancreatic duct, and a papilla can be exemplified.

In the ultrasound image recognition unit 168, the position and orientation detection unit 114 detects the position and the orientation of the distal end portion 40 of the ultrasound endoscope 12 from the ultrasound image for diagnosis based on the learning result.

Subsequently, the selection unit 116 selects a detection unit corresponding to the position of the distal end portion 40 of the ultrasound endoscope 12 detected by the position and orientation detection unit 114, from the organ name detection unit 112.

Subsequently, the organ name detection controller 118 performs control such that the detection unit selected by the selection unit 116 detects the name of the organ displayed in the ultrasound image for diagnosis from the ultrasound image for diagnosis based on the learning result.

Subsequently, the display controller 172 displays at least one of the name of the organ or the position of the distal end portion 40 of the ultrasound endoscope 12 recognized by the ultrasound image recognition unit 168, on the monitor 20. Alternatively, the display controller 172 displays at least one of the name of the organ recognized by the ultrasound image recognition unit 168 or the position and the orientation of the distal end portion 40 of the ultrasound endoscope 12 recognized by the ultrasound image recognition unit 168 similarly, on the monitor 20.

The display controller 172 superimposedly displays the lesion region on the endoscope image, superimposedly displays the name of the organ on the ultrasound image, or superimposedly displays the position and the orientation of the distal end portion 40 of the ultrasound endoscope 12 on the anatomical schema diagram in response to an instruction from the operator.

In other words, the display controller 172 displays one image or two or more images from among the endoscope image with the lesion region not displayed, the endoscope image with the lesion region superimposedly displayed, the ultrasound image with the name of the organ not displayed, the ultrasound image with the name of the organ superimposedly displayed, the anatomical schema diagram with the position of the distal end portion 40 of the ultrasound endoscope 12 not displayed, and the anatomical schema diagram with the position of the distal end portion 40 of the ultrasound endoscope 12 superimposedly displayed, in parallel within a screen of the monitor 20 in response to an instruction from the operator.

As an embodiment, the display controller 172 displays two or more images including at least one of the ultrasound image with the name of the organ superimposedly displayed or the anatomical schema diagram with the position of the distal end portion 40 of the ultrasound endoscope 12 superimposedly displayed, in parallel within the screen of the monitor 20.

The name of the organ is superimposedly displayed near the organ, for example, on the organ, for example, on the ultrasound image superimposedly, and the position and the orientation of the distal end portion 40 of the ultrasound endoscope 12 are superimposedly displayed, for example, on the anatomical schema diagram. The lesion region is superimposedly displayed, for example, on the endoscope image in a state in which the lesion region is surrounded by a frame line.

The cine memory 150 has a capacity for accumulating an image signal for one frame or image signals for several frames. An image signal generated by the ASIC 148 is output to the DSC 154, and is stored in the cine memory 150 by the memory controller 151. In a freeze mode, the memory controller 151 reads out the image signal stored in the cine memory 150 and outputs the image signal to the DSC 154. With this, an ultrasound image (static image) based on the image signal read from the cine memory 150 is displayed on the monitor 20.

The CPU 152 functions as a controller that controls the respective units of the ultrasound observation device 14. The CPU 152 is connected to the reception circuit 142, the transmission circuit 144, the A/D converter 146, the ASIC 148, and the like, and controls the equipment. Specifically, the CPU 152 is connected to the console 100, and controls the respective units of the ultrasound observation device 14 in compliance with examination information, control parameters, and the like input through the console 100.

In a case where the ultrasound endoscope 12 is connected to the ultrasound observation device 14 through the ultrasound connector 32a, the CPU 152 automatically recognizes the ultrasound endoscope 12 by a plug and play (PnP) system or the like.

### <<Operation Example of Ultrasound Endoscope System 10»

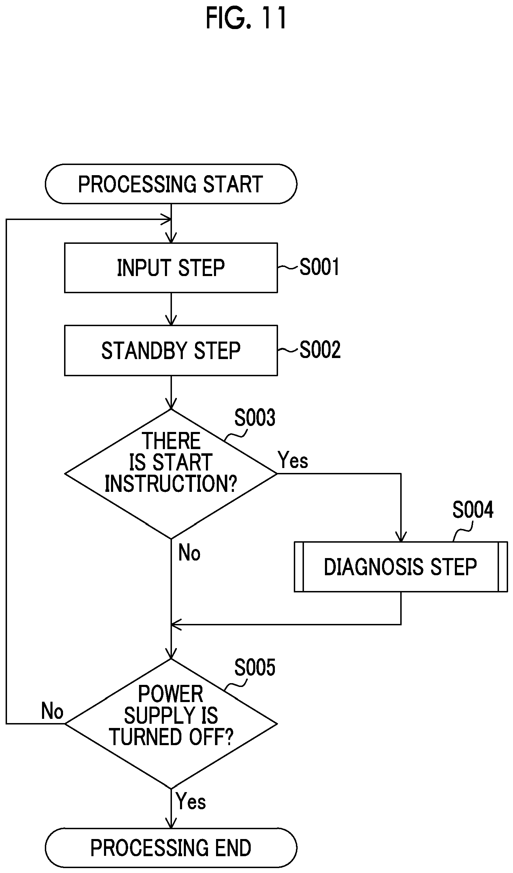

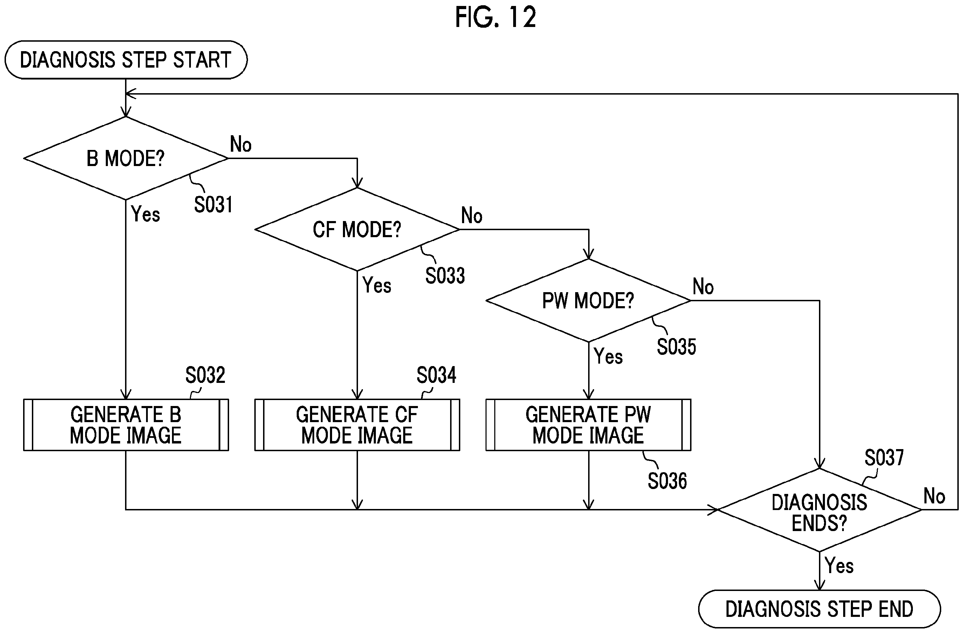

Next, as an operation example of the ultrasound endoscope system 10, a flow of a series of processing (hereinafter, also referred to as diagnosis processing) regarding ultrasonography will be described referring to Figs. 11 and 12. Fig. 11 is a flowchart showing a flow of diagnosis processing using the ultrasound endoscope system 10. Fig. 12 is a flowchart showing a procedure of a diagnosis step during the diagnosis processing.

In a case where power is supplied to the respective units of the ultrasound endoscope system 10 in a state in which the ultrasound endoscope 12 is connected to the ultrasound observation device 14, the endoscope processor 16, and the light source device 18, the diagnosis processing is started with the power supply as a trigger. In the diagnosis processing, as shown in Fig. 11, first, an input step is performed (S001). In the input step, the operator inputs the examination information, the control parameters, and the like through the console 100. In a case where the input step is completed, a standby step is performed until there is an instruction to start diagnosis (S002).

Subsequently, in a case where there is a diagnosis start instruction from the operator (in S003, Yes), the CPU 152 performs control on the respective units of the ultrasound observation device 14 to perform the diagnosis step (S004). The diagnosis step progresses along the flow shown in Fig. 12, and in a case where a designated image generation mode is a B mode (in S031, Yes), control is performed on the respective units of the ultrasound observation device 14 to generate a B mode image (S032). In a case where the designated image generation mode is not the B mode (in S031, No) but is a CF mode (in S033, Yes), control is performed on the respective units of the ultrasound observation device 14 to generate a CF mode image (S034). In a case where the designated image generation mode is not the CF mode (in S033, No) but is a PW mode (in S035, Yes), control is performed on the respective units of the ultrasound observation device 14 to generate a PW mode image (S036). In a case where the designated image generation mode is not the PW mode (in S035, No), the process progresses to Step S037.

Subsequently, the CPU 152 determines whether or not the ultrasonography ends (S037). In a case where the ultrasonography does not end (in S037, No), the process returns to the diagnosis step S031, and the generation of the ultrasound image in each image generation mode is repeatedly performed until a diagnosis end condition is established. As the diagnosis end condition, for example, a condition that the operator gives an instruction to end diagnosis through the console 100, or the like is exemplified.

On the other hand, in a case where diagnosis end condition is established and the ultrasonography ends (in S037, Yes), the diagnosis step ends.

Subsequently, returning to Fig. 11, in a case where the respective units of the ultrasound endoscope system 10 are powered-off (in S006, Yes), the diagnosis processing ends. On the other hand, in a case where the respective units of the ultrasound endoscope system 10 are maintained in a powered-on state (in S005, No), the process returns to the input step S001, and the respective steps of the diagnosis processing described above are repeated.

### <<Display Method of Endoscope Image, Ultrasound Image, and Anatomical Schema Diagram>>

Next, a display method of an endoscope image, an ultrasound image, and an anatomical schema diagram will be described.

The operator can display at least one of the endoscope image, the ultrasound image, or the anatomical schema diagram within the screen of the monitor 20 by operates the console 100 to give an instruction.

In this case, the display controller 172 displays one image or two or more images from among an endoscope image (with a lesion region displayed or not displayed), an ultrasound image (with a name of an organ displayed or not displayed), and the anatomical schema diagram (with a position and an orientation of the distal end portion 40 of the ultrasound endoscope 12 displayed or not displayed), in parallel within the monitor 20 in response to an instruction from the operator. The display controller 172 displays one image of the two or more images displayed on the monitor 20 as an image of interest to be greater than other images.

In the ultrasound endoscope system 10, the ultrasound image recognition unit 168 operates in a case where the ultrasound image or the anatomical schema diagram is displayed within the screen of the monitor 20, and the endoscope image recognition unit 170 operates in a case where the endoscope image is displayed within the screen of the monitor 20.

With this, in response to an instruction from the operator, it is possible to display the endoscope image with the lesion region superimposedly displayed, on the monitor 20, to display the ultrasound image with the name of the organ superimposedly displayed, on the monitor 20, or to display the anatomical schema diagram with the position and the orientation of the distal end portion 40 of the ultrasound endoscope 12 superimposedly displayed, on the monitor 20.

It is not essential to superimposedly display the name of the organ on the ultrasound image or to superimposedly display the position and the orientation of the distal end portion 40 of the ultrasound endoscope 12 on the anatomical schema diagram. For example, the name of the organ may be displayed on the monitor 20 separately from the ultrasound image, and the position and the orientation of the distal end portion 40 of the ultrasound endoscope 12 may be displayed on the monitor 20 separately from the anatomical schema diagram.

In the ultrasound endoscope system 10, the name of the organ displayed in the ultrasound image is displayed on the monitor 20 to be superimposed, for example, on the ultrasound image, and thus, for example, even an operator who is unaccustomed to an ultrasound image can correctly recognize what is displayed in the ultrasound image. The position and the orientation of the distal end portion 40 of the ultrasound endoscope 12 is displayed on the monitor 20 to be superimposed, for example, on the anatomical schema diagram, and thus, for example, even an operator who is unaccustomed to an ultrasound image can correctly recognize a position of the distal end portion 40 of the ultrasound endoscope 12, a direction of the distal end portion 40 of the ultrasound endoscope 12, and a part being observed at this moment, and does not get lost in the body of the subject. The lesion region is displayed on the monitor 20 to be superimposed on the endoscope image, and thus, it is possible to correctly recognize the lesion region.

For example, the operator can display the ultrasound image and the anatomical schema diagram in parallel within the screen of the monitor 20.

In this case, the display controller 172 displays the ultrasound image with the name of the organ superimposedly displayed and the anatomical schema diagram with the position and the orientation of the distal end portion 40 of the ultrasound endoscope 12 superimposedly displayed, in parallel within the screen of the monitor 20 in response to an instruction from the operator. Furthermore, one image of the ultrasound image and the anatomical schema diagram displayed on the monitor 20 is displayed as an image of interest to be greater than the other image.

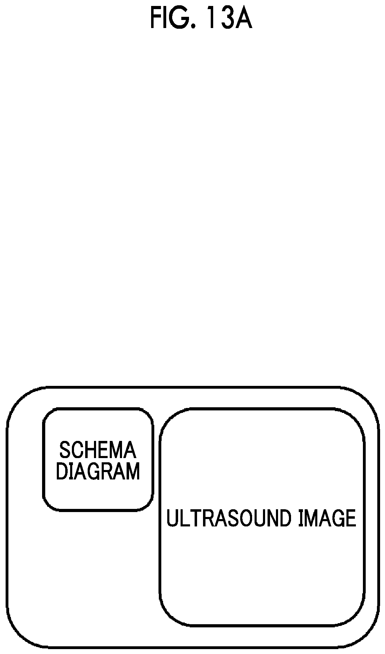

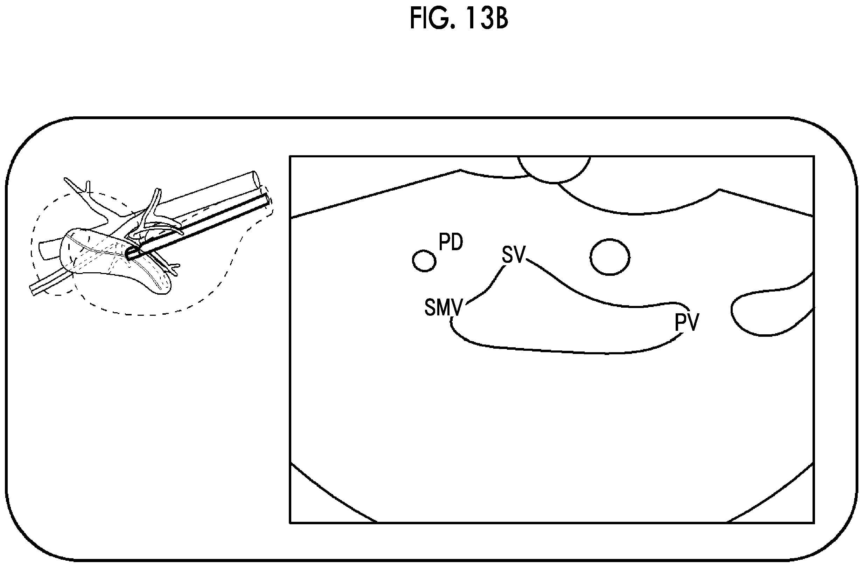

Fig. 13A is a conceptual diagram of an embodiment representing display positions of an ultrasound image and an anatomical schema diagram, and Fig. 13B is a conceptual diagram of an embodiment representing the ultrasound image and the anatomical schema diagram.

As shown in Figs. 13A and 13B, as an initial screen, for example, an anatomical schema diagram with the position and the orientation of the distal end portion 40 of the ultrasound endoscope 12 superimposedly displayed is displayed in an upper left portion within the screen of the monitor 20, and an ultrasound image with the name of the organ superimposedly displayed is displayed in a right portion within the screen of the monitor 20. Furthermore, as the initial screen, for example, the ultrasound image is displayed to be greater than the anatomical schema diagram.

The operator can display an ultrasound image (first ultrasound image for diagnosis) with the name of the organ superimposedly displayed, an ultrasound image (second ultrasound image for diagnosis) that is the same ultrasound image as the first ultrasound image for diagnosis and on which the name of the organ is not displayed, and an anatomical schema diagram with the position and the orientation of the distal end portion 40 of the ultrasound endoscope 12 superimposedly displayed, within the screen of the monitor 20.

In this case, the display controller 172 displays, for example, the first ultrasound image for diagnosis, the second ultrasound image for diagnosis, and the anatomical schema diagram with the position and the orientation of the distal end portion 40 of the ultrasound endoscope 12 superimposedly displayed, in parallel within the screen of the monitor 20 in response to an instruction from the operator. Furthermore, one image of the first ultrasound image for diagnosis, the second ultrasound image for diagnosis, and the anatomical schema diagram displayed on the monitor 20 is displayed as an image of interest to be greater than other images.

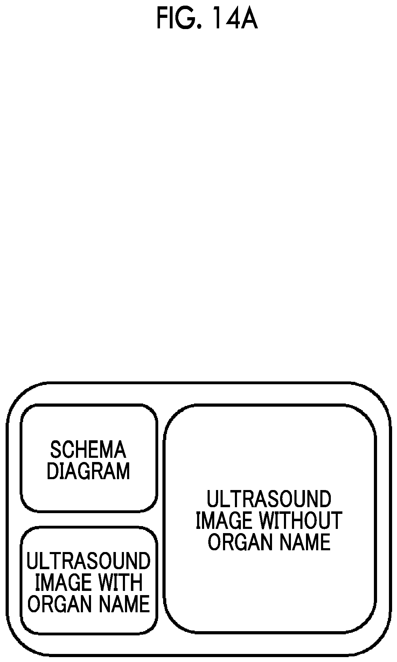

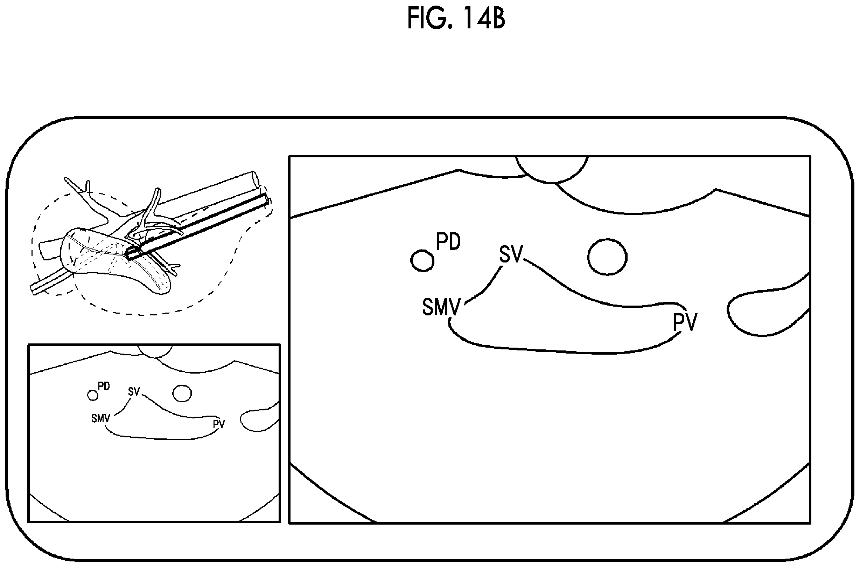

Fig. 14A is a conceptual diagram of an embodiment representing display positions of a first ultrasound image for diagnosis, a second ultrasound image for diagnosis, and an anatomical schema diagram, and Fig. 14B is a conceptual diagram of an embodiment representing the first ultrasound image for diagnosis, the second ultrasound image for diagnosis, and the anatomical schema diagram.

As shown in Figs. 14A and 14B, as an initial screen, for example, the anatomical schema diagram with the position and the orientation of the distal end portion 40 of the ultrasound endoscope 12 superimposedly displayed is displayed in an upper left portion within the screen of the monitor 20, the first ultrasound image for diagnosis is displayed in a lower left portion within the screen of the monitor 20, and the second ultrasound image for diagnosis is displayed in a right portion within the screen of the monitor 20. Furthermore, as the initial screen, for example, the second ultrasound image for diagnosis is displayed to be greater than the anatomical schema diagram and the first ultrasound image for diagnosis.

The operator can display an endoscope image, an ultrasound image, and the anatomical schema diagram within the screen of the monitor 20.

In this case, the display controller 172 displays, for example, the endoscope image, the ultrasound image with the name of the organ superimposedly displayed, and the anatomical schema diagram with the position and the orientation of the distal end portion 40 of the ultrasound endoscope 12 superimposedly displayed, in parallel within the screen of the monitor 20 in response to an instruction from the operator. Furthermore, one image of the endoscope image, the ultrasound image, and the anatomical schema diagram displayed on the monitor 20 is displayed as an image of interest to be greater than other images.

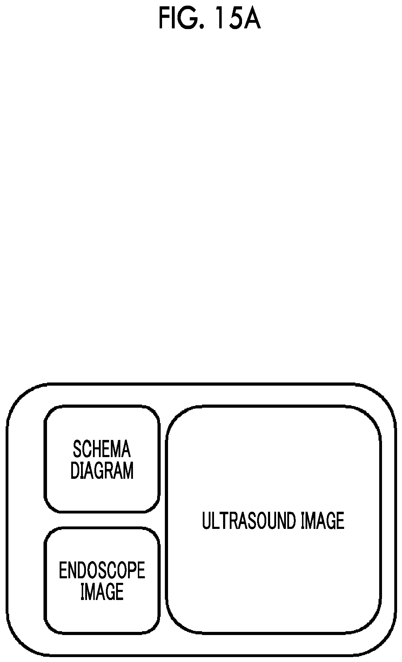

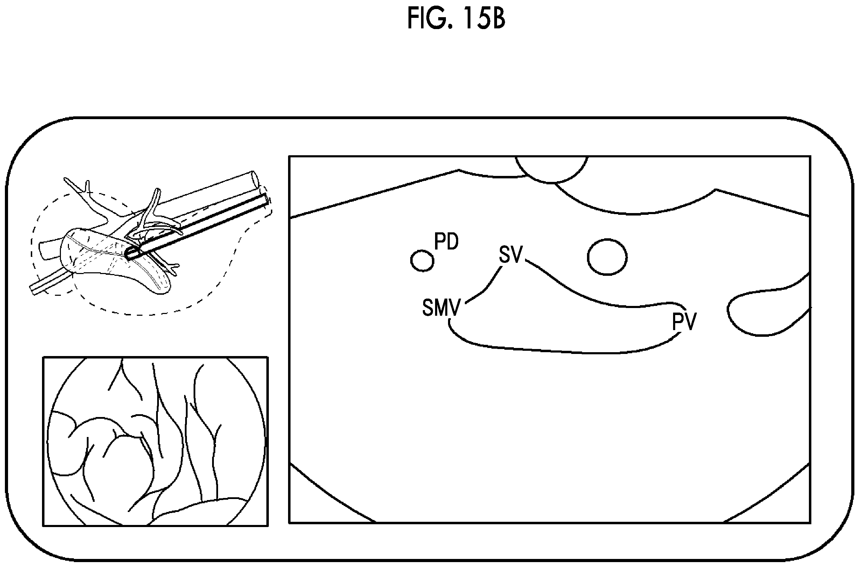

Fig. 15A is a conceptual diagram of an embodiment representing display positions of an endoscope image, an ultrasound image, and an anatomical schema diagram, and Fig. 15B is a conceptual diagram of an embodiment representing the endoscope image, the ultrasound image, and the anatomical schema diagram.

As shown in Figs. 15A and 15B, as an initial screen, for example, the anatomical schema diagram with the position and the orientation of the distal end portion 40 of the ultrasound endoscope 12 superimposedly displayed is displayed in an upper left portion within the screen of the monitor 20, the endoscope image with the lesion region not displayed is displayed in a lower left portion within the screen of the monitor 20, and the ultrasound image with the name of the organ superimposedly displayed is displayed in a right portion within the screen of the monitor 20. Furthermore, as the initial screen, for example, the ultrasound image is displayed to be greater than the anatomical schema diagram and the endoscope image.

Although a display method of an endoscope image, an ultrasound image, and the anatomical schema diagram has been described, the invention is not limited thereto, one image can be displayed within the screen of the monitor 20 or two or more images can be arbitrarily combined and displayed in parallel within the screen of the monitor 20.

As the initial screen, the positions where the endoscope image, the ultrasound image, and the anatomical schema diagram are disposed, and one image displayed to be greater than other images from among the images displayed on the monitor 20 can be arbitrarily set. For example, as the initial image, the first ultrasound image for diagnosis and the second ultrasound image for diagnosis shown in Figs. 14A and 14B may be switched and displayed.

The operator can switch and display an image of interest from among the images displayed on the monitor 20.

For example, it is assumed that an endoscope image, an ultrasound image, and an anatomical schema diagram are displayed within the screen of the monitor 20.

In this case, the display controller 172 switches and displays an image of interest from one image from among the endoscope image, the ultrasound image, and the anatomical schema diagram displayed on the monitor 20 to one of other images in response to an instruction from the operator.

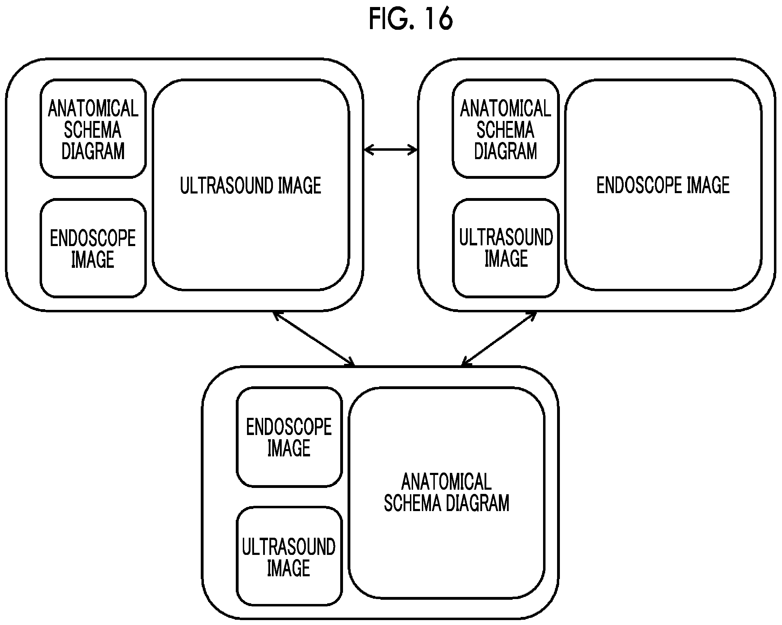

As shown in an upper left portion of Fig. 16, as an initial screen, it is assumed that an anatomical schema diagram is displayed in an upper left portion within the screen of the monitor 20, an endoscope image is displayed in a lower left portion within the screen of the monitor 20, and an ultrasound image is displayed in a right portion within the screen of the monitor 20. Furthermore, as the initial screen, it is assumed that the ultrasound image is displayed as an image of interest to be greater than the anatomical schema diagram and the endoscope image.

For example, in a case where the endoscope image is selected as an image of interest by the operator from the state in the upper left portion of Fig. 16, as shown in an upper right portion of Fig. 16, the anatomical schema diagram is displayed in the upper left portion within the screen of the monitor 20, the ultrasound image is displayed in the lower left portion within the screen of the monitor 20, and the endoscope image is displayed in the right portion within the screen of the monitor 20. The endoscope image is displayed to be greater than the anatomical schema diagram and the ultrasound image.

In a case where the anatomical schema diagram is selected as an image of interest by the operator from the state in the upper left portion of Fig. 16, as shown in a lower portion of Fig. 16, the endoscope image is displayed in the upper left portion within the screen of the monitor 20, the ultrasound image is displayed in the lower left portion within the screen of the monitor 20, and the anatomical schema diagram is displayed in the right portion within the screen of the monitor 20. The anatomical schema diagram is displayed to be greater than the endoscope image and the ultrasound image.

An operation in a case where the anatomical schema diagram is selected as an image of interest by the operator from the state in the upper right portion of Fig. 16 is also the same as the operation in a case where the anatomical schema diagram is selected as an image of interest from the state in the upper left portion of Fig. 16.

In a case where the ultrasound image is selected as an image of interest by the operator from the state in the upper right portion of Fig. 16, as shown in the upper left portion of Fig. 16, the ultrasound image is displayed to be greater than the anatomical schema diagram and the endoscope image.

An operation in a case where the ultrasound image is selected as an image of interest by the operator from the state in the lower portion of Fig. 16 is also the same as the operation in a case where the ultrasound image is selected as an image of interest from the state in the upper right portion of Fig. 16.

In the ultrasound endoscope system 10, it is possible to switch and display an endoscope image, an ultrasound image, and an anatomical schema diagram easily to see. While an image of interest in which the operator is interested changes occasionally, the operator can switch the image of interest at any timing, and thus, it is possible to allow the operator to display and view an image in which the user is interested on the occasion, as an image of interest to be greater than other images.

Although an example where an image of interest is switched among an endoscope image, an ultrasound image, and an anatomical schema diagram displayed on the monitor 20 has been described, the invention is not limited thereto, and the same operation is performed even in a case where an image of interest is switched between two or more images displayed on the operation monitor 20.

### <<Disposition Places of Ultrasound Image Recognition Unit 168 and Endoscope Image Recognition Unit 170>>

Next, disposition places of the ultrasound image recognition unit 168 and the endoscope image recognition unit 170 will be described.

In the embodiment, although the ultrasound image recognition unit 168 is incorporated in the ultrasound observation device 14, the invention is not limited thereto, and the ultrasound image recognition unit 168 may be incorporated, for example, in the endoscope processor 16 or may be provided outside the ultrasound observation device 14 and the endoscope processor 16.

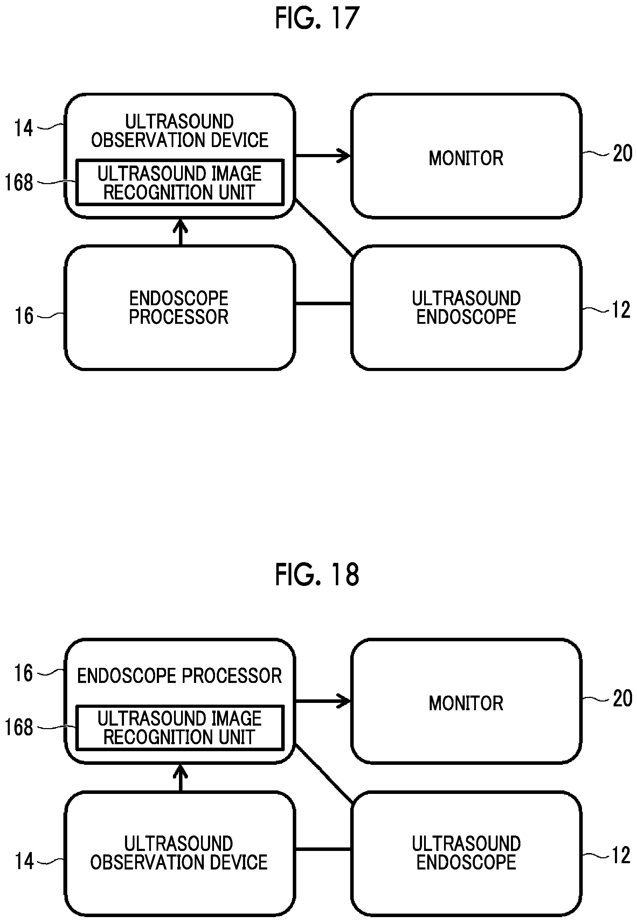

As in the embodiment, in a case where the ultrasound image recognition unit 168 is incorporated in the ultrasound observation device 14, as shown in Fig. 17, an endoscope image is transferred from the endoscope processor 16 to the ultrasound observation device 14.

In a case where the ultrasound image recognition unit 168 is incorporated in the endoscope processor 16, as shown in Fig. 18, an ultrasound image is transferred from the ultrasound observation device 14 to the endoscope processor 16.

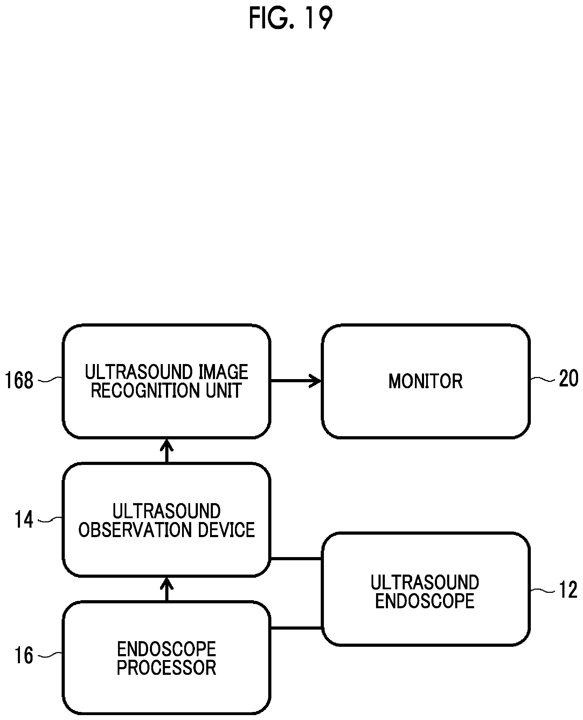

In a case where the ultrasound image recognition unit 168 is provided outside the ultrasound observation device 14 and the endoscope processor 16, as shown in Fig. 19, an endoscope image is transferred from the endoscope processor 16 to the ultrasound observation device 14, and the endoscope image and an ultrasound image are further transferred from the ultrasound observation device 14 to the ultrasound image recognition unit 168.

In this case, the ultrasound image may be transferred from the ultrasound observation device 14 to the endoscope processor 16, and the endoscope image and the ultrasound image may be further transferred from the endoscope processor 16 to the ultrasound image recognition unit 168. Alternatively, the endoscope image may be transferred from the endoscope processor 16 to the ultrasound observation device 14, and may be further transferred from the endoscope processor 16 to the ultrasound image recognition unit 168 instead of being transferred from the ultrasound observation device 14 to the ultrasound image recognition unit 168.

The display controller 172 is disposed between a final image signal that is output to the monitor 20, and the monitor 20.

In a case where the ultrasound image recognition unit 168 is incorporated in the ultrasound observation device 14, the display controller 172 can be incorporated, for example, in the ultrasound observation device 14 or can be provided between the ultrasound observation device 14 and the monitor 20.

In a case where the ultrasound image recognition unit 168 is incorporated in the endoscope processor 16, the display controller 172 can be incorporated, for example, in the endoscope processor 16 or can be provided between the endoscope processor 16 and the monitor 20.

In a case where the ultrasound image recognition unit 168 is provided outside the ultrasound observation device 14 and the endoscope processor 16, the display controller 172 can be provided, for example, outside the ultrasound observation device 14 and the endoscope processor 16.

The display controller 172 displays one image or two or more images from among the endoscope image (with the lesion region displayed or not displayed), the ultrasound image (with the name of the organ displayed or not displayed), and the anatomical schema diagram (with the position and the orientation of the distal end portion 40 of the ultrasound endoscope 12 displayed or not displayed), in parallel within the screen of the monitor 20 in response to an instruction from the operator.

The disposition place of the endoscope image recognition unit 170 can be decided in the same manner as the disposition place of the ultrasound image recognition unit 168. That is, in the embodiment, although the endoscope image recognition unit 170 is incorporated in the endoscope processor 16, the invention is not limited thereto, and endoscope image recognition unit 170 may be incorporated, for example, in the ultrasound observation device 14 or may be provided outside the ultrasound observation device 14 and the endoscope processor 16.

In this way, in the ultrasound endoscope system 10, the disposition places of the ultrasound image recognition unit 168 and the endoscope image recognition unit 170 are not fixed, and the ultrasound image recognition unit 168 and the endoscope image recognition unit 170 can be provided at any disposition places.

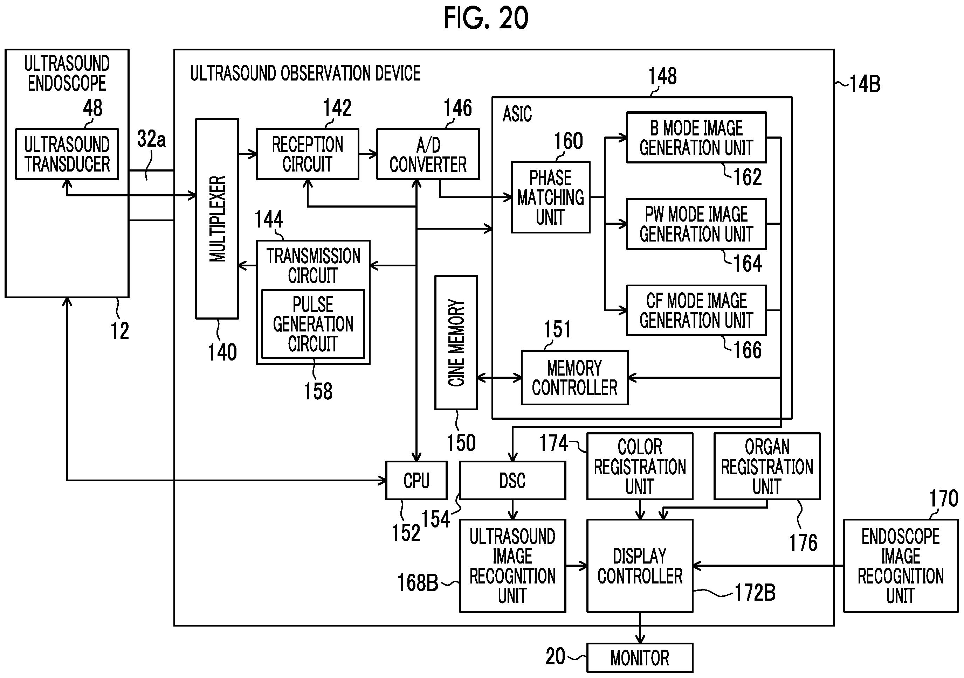

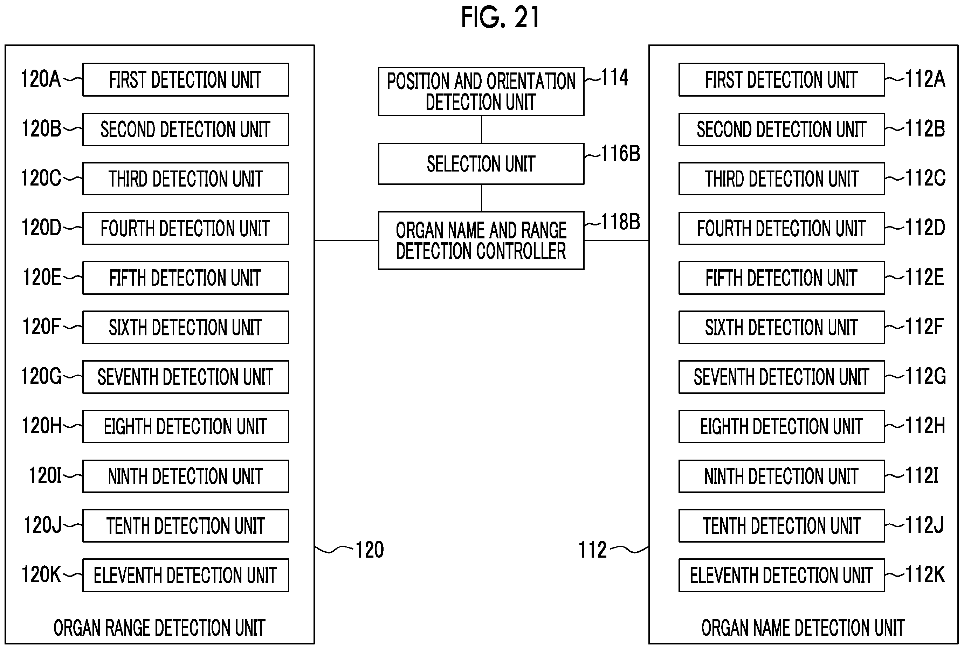

Next, an ultrasound endoscope system according to a second embodiment will be described referring to Figs. 20 and 21. Fig. 20 is a block diagram showing the configuration of an ultrasound observation device 14B of the second embodiment, and Fig. 21 is a block diagram showing the configuration of an ultrasound image recognition unit 168B of the second embodiment.

### «Outline of Ultrasound Endoscope System»

The configuration of the ultrasound endoscope system of the second embodiment is the same as the configuration of the ultrasound endoscope system 10 of the first embodiment except that the ultrasound observation device 14B is provided instead of the ultrasound observation device 14 provided in the ultrasound endoscope system 10 of the first embodiment, and thus, detailed description of other identical components will not be repeated.

Hereinafter, the ultrasound observation device 14B will be described.

### <<Configuration of Ultrasound Observation Device 14B>>

The configuration of the ultrasound observation device 14B shown in Fig. 20 is the same as the configuration of the ultrasound observation device 14 of the first embodiment except that an ultrasound image recognition unit 168B and a display controller 172B are provided instead of the ultrasound image recognition unit 168 and the display controller 172 provided in the ultrasound observation device 14 of the first embodiment, and a color registration unit 174 and an organ registration unit 176. Thus, other identical components are represented by identical reference numerals, and detailed description thereof will not be repeated.

The ultrasound image recognition unit 168B functions in the same manner as the ultrasound image recognition unit 168 of the first embodiment in regards to the learning and the recognition of the name of the organ displayed in the ultrasound image for diagnosis and the position and the orientation of the distal end portion 40 of the ultrasound endoscope 12.

In addition, the ultrasound image recognition unit 168B learns a relationship between an ultrasound image for learning and a range of an organ (a region of an organ) displayed in the ultrasound image for learning in advance on a plurality of ultrasound images for learning, and recognizes a range of an organ displayed in an ultrasound image for diagnosis from the ultrasound image for diagnosis based on a learning result.

The ultrasound image for learning is an existing ultrasound image that is used for the ultrasound image recognition unit 168B to learn a relationship between an ultrasound image and a range of an organ, and for example, various ultrasound images captured in the past can be used.

The configuration of the ultrasound image recognition unit 168B shown in Fig. 21 is the same as the configuration of the ultrasound image recognition unit 168 of the first embodiment, except that a selection unit 116B and an organ name and range detection controller 118B are provided instead of the selection unit 116 and the organ name detection controller 118 provided in the ultrasound image recognition unit 168 of the first embodiment, and an organ range detection unit 120 is further provided. Thus, other identical components are represented by identical reference numerals, and detailed description thereof will not be repeated.

The organ range detection unit 120 detects a range of an organ displayed in an ultrasound image for diagnosis from the ultrasound image for diagnosis based on a learning result. The organ range detection unit 120 comprises a plurality of detection units corresponding to a plurality of positions each to be an observation target part in the body of the subject. Here, as an example, the organ range detection unit 120 comprises first to eleventh detection units 120A to 120K. The first detection unit 120A corresponds to a confluence of an aorta, a celiac artery, and a superior mesenteric artery, the second detection unit 120B corresponds to a pancreatic body, the third detection unit 120C corresponds to a pancreatic tail, the fourth detection unit 120D corresponds to a confluence of a splenic vein, a superior mesenteric vein, and a portal vein, the fifth detection unit 120E corresponds to a pancreatic head, the sixth detection unit 120F corresponds to a gallbladder, the seventh detection unit 120G corresponds to a portal vein, the eighth detection unit 120H corresponds to a common bile duct, the ninth detection unit 1201 corresponds to a gallbladder, the tenth detection unit 120J corresponds to a pancreatic uncinate process, and the eleventh detection unit 120K corresponds to a papilla.

The first to eleventh detection units 120A to 120K are learned models. A plurality of learned models are models learned using respective data sets having different ultrasound images for learning. In detail, a plurality of learned models are models learned a relationship between an ultrasound image for learning and a range of an organ displayed in the ultrasound image for learning in advance using data sets having ultrasound images for learning obtained by imaging different positions each to be an observation target part in the body of the subject.

That is, the first detection unit 120A is a model learned using a data set having ultrasound images for learning of the confluence of the aorta, the celiac artery, and the superior mesenteric artery, the second detection unit 120B is a model learned using a data set having ultrasound images for learning of the pancreatic body, the third detection unit 120C is a model learned using a data set having ultrasound images for learning of the pancreatic tail, the fourth detection unit 120D is a model learned using a data set having ultrasound images for learning of the confluence of the splenic vein, the superior mesenteric vein, and the portal vein, the fifth detection unit 120E is a model learned using a data set having ultrasound images for learning of the pancreatic head, the sixth detection unit 120F is a model learned using a data set having ultrasound images for learning of the gallbladder, the seventh detection unit 120G is a model learned using a data set having ultrasound images for learning of the portal vein, the eighth detection unit 120H is a model learned using a data set having ultrasound images for learning of the common bile duct, the ninth detection unit 120I is a model learned using a data set having ultrasound images for learning of the gallbladder, the tenth detection unit 120J is a model learned using a data set having ultrasound images for learning of the pancreatic uncinate process, the eleventh detection unit 120K is a model learned using a data set having ultrasound images for learning of the papilla.

As described above, the observation route in the body in a case of capturing the ultrasound image and the representative observation points are generally determined. For example, it is possible to learn an ultrasound image at a representative observation point and a range of an organ displayed in the ultrasound image in association with each other.

A learning method is not particularly limited as long as it is possible to learn the relationship between the ultrasound image and the range of the organ from a plurality of ultrasound images for learning, and to generate a learned model. An update method and the like of the learning method and the learned model are as described above.

The selection unit 116B functions in the same manner as the selection unit 116 of the first embodiment in regard to the selection of the detection unit from the organ name detection unit 112.

In addition, the selection unit 116B selects a detection unit corresponding to the position of the distal end portion 40 of the ultrasound endoscope 12 detected by the position and orientation detection unit 114, from the organ range detection unit 120.

That is, the selection unit 116B selects the first detection unit 120A in a case where the position of the distal end portion 40 of the ultrasound endoscope 12 is (1) confluence of aorta, celiac artery, and superior mesenteric artery, selects the second detection unit 120B in a case where the position of the distal end portion 40 of the ultrasound endoscope 12 is (2) pancreatic body, selects the third detection unit 120C in a case where the position of the distal end portion 40 of the ultrasound endoscope 12 is (3) pancreatic tail, selects the fourth detection unit 120D in a case where the position of the distal end portion 40 of the ultrasound endoscope 12 is (4) confluence of splenic vein, superior mesenteric vein, and portal vein, selects the fifth detection unit 120E in a case where the position of the distal end portion 40 of the ultrasound endoscope 12 is (5) pancreatic head, selects the sixth detection unit 120F in a case where the position of the distal end portion 40 of the ultrasound endoscope 12 is (6) gallbladder, selects the seventh detection unit 120G in a case where the position of the distal end portion 40 of the ultrasound endoscope 12 is (7) portal vein, selects the eighth detection unit 120H in a case where the position of the distal end portion 40 of the ultrasound endoscope 12 is (8) common bile duct, selects the ninth detection unit 120I in a case where the position of the distal end portion 40 of the ultrasound endoscope 12 is (9) gallbladder, selects the tenth detection unit 120J in a case where the position of the distal end portion 40 of the ultrasound endoscope 12 is (10) pancreatic uncinate process, and selects the eleventh detection unit 120K in a case where the position of the distal end portion 40 of the ultrasound endoscope 12 is (11) papilla.

The organ name and range detection controller 118B functions in the same manner as the organ name detection controller 118 of the first embodiment in regard to the control of the organ name detection unit 112.

In addition, the organ name and range detection controller 118B makes the detection unit selected by the selection unit 116B from the organ range detection unit 120 detect a range or an organ displayed in an ultrasound image for diagnosis from the ultrasound image for diagnosis.

As the organ having the range detected by the organ range detection unit 120, all observation target parts in the body of the subject that can be observed using the ultrasound observation device 14 are included.

In the ultrasound image recognition unit 168B, the position and orientation detection unit 114 detects the position and the orientation of the distal end portion 40 of the ultrasound endoscope 12 from the ultrasound image for diagnosis based on the learning result.

Subsequently, the selection unit 116B selects a detection unit corresponding to the position of the distal end portion 40 of the ultrasound endoscope 12 detected by the position and orientation detection unit 114, from the organ name detection unit 112 and the organ range detection unit 120.

Subsequently, the organ name and range detection controller 118B performs control such that the detection unit selected by the selection unit 116B detects the name of the organ displayed in the ultrasound image for diagnosis and the range of the organ from the ultrasound image for diagnosis based on the learning result.

In the ultrasound endoscope system of the second embodiment, for example, the name of the organ displayed in the ultrasound image and the range of the organ are displayed on the monitor 20 to be superimposed on the ultrasound image, and the position and the orientation of the distal end portion 40 of the ultrasound endoscope 12 are displayed on the monitor 20 to be superimposed on the anatomical schema diagram. For this reason, for example, even an operator who is unaccustomed to an ultrasound image can correctly recognize what is displayed in the ultrasound image and a range of an organ displayed in the ultrasound image. Furthermore, the operator can correctly recognize the position of the distal end portion 40 of the ultrasound endoscope 12, the direction of the distal end portion 40 of the ultrasound endoscope 12, and a part being observed, and does not get lost in the body of the subject.

Returning to Fig. 20, the display controller 172B functions in the same manner as the display controller 172 of the first embodiment.

In addition, the display controller 172B displays the range of the organ recognized by the ultrasound image recognition unit 168B, on the monitor 20.

The color registration unit 174 registers a relationship between a type of an organ and a color of a range of an organ in response to an instruction from the operator. In more detail, a relationship between a type of an organ and a color of an internal region of a range of an organ or a frame indicating the range of the organ is registered. The relationship between the type of the organ and the color of the internal region or the frame of the organ registered in the color registration unit 174 is output to the display controller 172B.

The frame (hereinafter, referred to as the frame of the organ) indicating the range of the organ is a contour of the organ, and is a boundary line between the organ and another organ.

The internal region of the range of the organ (hereinafter, referred to as the internal region of the organ) is a region in a closed space surrounded by the frame of the organ.

The organ registration unit 176 registers a type of an organ for displaying a range in response to an instruction from the operator. The type of the organ for displaying the range registered in the organ registration unit 176 is output to the display controller 172B.

### «Display Method of Name of Organ and Range of Organ»

Next, a display method of the name of the organ and the range of the organ will be described.

The operator can designate whether or not to display a range of an organ displayed in an ultrasound image.

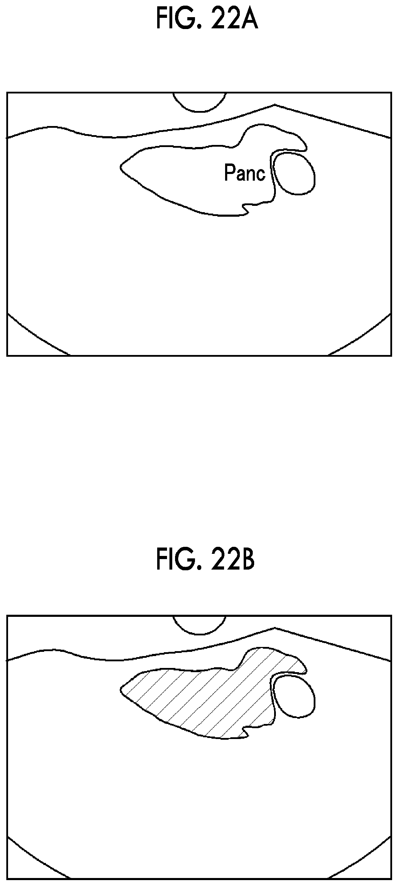

In a case where designation is made to display the range of the organ in response to an instruction from the operator, as shown in Fig. 22B, the display controller 172B colors, for example, the internal region of the range of the organ recognized by the ultrasound image recognition unit 168B in a given color, and the range of the organ with the internal region colored is displayed on the monitor 20. Alternatively, a frame indicating the range of the organ recognized by the ultrasound image recognition unit 168B is provided, the frame is colored in a given color, and the range of the organ with the frame colored is displayed on the monitor 20.

On the other hand, in a case where designation is made not to display the range of the organ in response to an instruction from the operator, the display controller 172B does not display the range of the organ.

The internal region or the frame of the organ is colored and displayed, whereby it is possible to allow the operator to clearly recognize the range of the organ.

The given color is a color that is set in advance in the display controller 172B, and is not particularly limited but is desirably a color other than white or black such that the operator easily recognizes the range of the organ in the ultrasound image.

It is desirable that the display controller 172B colors the internal region or the frame of the organ for each type of the organ having the range recognized by the ultrasound image recognition unit 168B, in a different color.

The display controller 172B can color, for example, the internal regions or the frames of two or more adjacent organs in colors having hue at equal intervals or colors in a given hue range including colors having hue at equal intervals such that the operator easily identifies a difference in color. For example, the display controller 172B can color an internal region or a frame of a blood vessel, an internal region or a frame of a vessel in which a body fluid other than blood flows, and an internal region or a frame of an organ other than the blood vessel and the vessel in colors having hue at equal intervals or colors in a given hue range including colors having hue at equal intervals similarly.

For example, in a case where two organs are adjacent, an internal region or a frame of one organ is colored in a complementary color of a color of an internal region or a frame of the other organ or a color in a given hue range including the complementary color. In a case where three organs are adjacent, internal regions or frames of the three organs are colored in colors having hue at equal intervals, such as red (R), green (G), and blue (B).

With this, even though two or more organs are adjacent, the operator can clearly recognize the range of each organ based on the difference in color.

The operator can color a range of an organ in a color designated by the operator or can color a range of an organ in a color registered in advance by the operator.

In a case where a color for coloring a range of an organ is designated in response to an instruction from the operator, the display controller 172B colors the internal region or the frame of the organ in the color designated by the instruction from the operator. Alternatively, in a case where a relationship between a type of an organ and a color of an internal region or a frame of an organ is registered in the color registration unit 174, the display controller 172B colors the internal region or the frame of the organ in a color of an internal region or a frame of an organ corresponding to the type of the organ registered in the color registration unit 174.

In this way, the operator can color the range of the organ in a color desired by the operator, and thus, for example, it is possible to the operator to easily identify a type of an organ based on a color in such a manner that red is a liver, for example.

In a case of detecting the name of the organ displayed in the ultrasound image for diagnosis, the organ name detection unit 112 of the ultrasound image recognition unit 168B can calculate a confidence factor of the name of the organ recognized by the organ name detection unit 112.

The confidence factor of the name of the organ represents a probability that the name of the organ recognized by the organ name detection unit 112 is a correct name. For example, in a case where detection is made that the name of the organ is a liver, the confidence factor of the name of the organ is calculated in such a manner that the probability that the name of the organ displayed in the ultrasound image for diagnosis is a liver is 90%.

In this case, the display controller 172B can decide at least one of the display method of the name of the organ or the coloring method of the internal region or the frame of the organ displayed on the monitor 20 depending on the confidence factor calculated by the ultrasound image recognition unit 168B.

In a case of displaying the name of the organ, the display controller 172B can perform at least one of, for example, an operation to decide the size of the name of the organ displayed on the monitor 20, an operation to decide the color for coloring the internal region or the frame of the organ, or an operation to decide whether or not to display a specific character, depending on the confidence factor.

For example, in a case where the confidence factor is comparatively low, the display controller 172B decreases the size of the name of the organ, decreases the density of the color for coloring the internal region or the frame of the organ, or displays the specific character, such as "?", to express that the probability that the name of the organ is a liver is comparatively low, like "liver?" in a case where it is assumed that the name of the organ is a liver, compared to a case where the confidence factor is comparatively high.

In a case of coloring the internal region or the frame of the organ, the display controller 172B can decide, for example, the density of the color for coloring the internal region or the frame of the organ depending on the confidence factor.

For example, in a case where the confidence factor is comparatively low, the density of the color for coloring the internal region or the frame of the organ decreases, and in a case where the confidence factor is comparatively higher, the density of the color for coloring the internal region or the frame of the organ increases.

In this way, the display method of the name of the organ and the coloring method of the internal region or the frame of the organ are decided, whereby it is possible to allow the operator to determine whether or not the name of the organ recognized by the ultrasound image recognition unit 168B is correct.

In a case where the name of the organ is superimposedly displayed on the ultrasound image for diagnosis, it is desirable that the display controller 172B decides at least one of a color of the name of the organ or the color of the internal region or the frame of the organ depending on the brightness of the ultrasound image for diagnosis displayed behind a display region of the name of the organ.

In a case where the brightness of the ultrasound image behind the display region of the name of the organ is comparatively high, the display controller 172B increases the density of the color of the name of the organ or decreases the density of the color of the internal region or the frame of the organ.

With this, even though the name of the organ and the ultrasound image in the background are superimposed or the name of the organ and the colored internal region or the frame of the organ in the background are superimposed, it is possible to allow the operator to easily see the name of the organ.

The operator can designate whether or not to display the name of the organ and whether or not to color the range of the organ.

The display controller 172B switches whether to display only one, both, or none of the name of the organ recognized by the ultrasound image recognition unit 168B and the range of the organ with the internal region or the frame colored, in response to an instruction from the operator.

For example, in a case where designation is made to display only the name of the organ in response to an instruction from the operator, as shown in Fig. 22A, the display controller 172B displays only the name of the organ without coloring the range of the organ. In a case where designation is made to perform only coloring of the range of the organ, as shown in Fig. 22B, the display controller 172B colors only the range of the organ without displaying the name of the organ. In a case where designation is made to display the name of the organ and to perform coloring of the range of the organ, the name of the organ is displayed, and the range of the organ is colored. On the other hand, in a case where designation is made not to display the name of the organ and not to perform coloring of the range of the organ, the name of the organ is not displayed, and the range of the organ is colored.

The display controller 172B can determine a position where the name of the organ recognized by the ultrasound image recognition unit 168B is displayed on the monitor 20 or can determine whether or not to display the name of the organ on the monitor 20, depending on the range of the organ recognized by the ultrasound image recognition unit 168B.

The display controller 172B does not display the name of the organ, for example, in a case where the range of the organ recognized by the ultrasound image recognition unit 168B is comparatively small, and displays the name of the organ in a case where the range of the organ is comparatively large. In a case where the range of the organ is comparatively small, the name of the organ is displayed near the range of the organ, not within the range of the organ, and in a case where the range of the organ is comparatively large, the name of the organ is displayed within the range of the organ.

The operator can display a range of only an organ of a type registered in advance by the operator.

In a case where a type of an organ for displaying a range is registered in the organ registration unit 176, only in a case where an organ having a range recognized by the ultrasound image recognition unit 168B is an organ registered in the organ registration unit 176, the display controller 172B displays the range of the organ recognized by the ultrasound image recognition unit 168B, on the monitor 20.

In this way, the operator can display a range of only an organ of a desired type, whereby it is possible to allow the operator to easily recognize the organ of the desired type.

The operator can designate a type of an organ for displaying a range.

For example, in a case where the type of the organ for displaying the range is sequentially designated in response to an instruction from the operator, the display controller 172B sequentially switches the type of the organ for displaying the range accordingly.

In the device of the invention, for example, the hardware configurations of processing units that execute various kinds of processing, such as the endoscope image recognition unit 170 (the lesion region detection unit 102, the positional information acquisition unit 104, the selection unit 106, and the lesion region detection controller 108), the ultrasound image recognition units 168 and 168B (the organ name detection unit 112, the organ range detection unit 120, the position and orientation detection unit 114, the selection unit 116, the selection unit 116B, the organ name detection controller 118, and the organ name and range detection controller 118B), the display controllers 172 and 172B, and the console (instruction acquisition unit) 100 may be dedicated hardware or may be various processors or computers that execute programs. The hardware configurations of the cine memory 150, the color registration unit 174, and the organ registration unit 176 may be dedicated hardware or may be a memory, such as a semiconductor memory.

Various processors include a central processing unit (CPU) that is a general-purpose processor executing software (program) to function as various processing units, a programmable logic device (PLD) that is a processor capable of changing a circuit configuration after manufacture, such as a field programmable gate array (FPGA), a dedicated electric circuit that is a processor having a circuit configuration dedicatedly designed for executing specific processing, such as an application specific integrated circuit (ASIC), and the like.

One processing unit may be configured of one of various processors described above or may be configured of a combination of two or more processors of the same type or different types, for example, a combination of a plurality of FPGAs, a combination of an FPGA and a CPU, or the like. Furthermore, a plurality of processing units may be configured of one among various processors or may be configured using one processor obtained by combining two or more of a plurality of processing units.

For example, as represented by a computer, such as a server or a client, there is a form in which one processor is configured of a combination of one or more CPUs and software, and the processor functions as a plurality of processing units. For example, as represented by system on chip (SoC) or the like, there is a form in which a processor that implements all functions of a system including a plurality of processing units into one integrated circuit (IC) chip is used.

In addition, the hardware configuration of various processors is, more specifically, an electric circuit (circuitry), in which circuit elements, such as semiconductor elements, are combined.

For example, a method according to the embodiment of the invention can be implemented by a program that causes a computer to execute respective steps. Furthermore, it is possible to provide a computer-readable recording medium having the program recorded thereon.

Although the invention has been described above in detail, the invention is not limited to the above-described embodiment, and various improvements and alterations may be of course made without departing the scope of the invention.

### Explanation of References

10: ultrasound endoscope system
12: ultrasound endoscope
14, 14B: ultrasound observation device
16: endoscope processor
18: light source device
20: monitor
21a: water supply tank
21b: suction pump
22: insertion part
24: operating part
26: universal cord
28a: air and water supply button
28b: suction button
29: angle knob
30: treatment tool insertion port
32a: ultrasound connector
32b: endoscope connector
32c: light source connector
34a: air and water supply tube
34b: suction tube
36: ultrasound observation part
38: endoscope observation part
40: distal end portion
42: bending portion
43: flexible portion
44: treatment tool lead-out port
45: treatment tool channel
46: ultrasound transducer unit
48: ultrasound transducer
50: ultrasound transducer array
54: backing material layer
56: coaxial cable
60: FPC
74: acoustic matching layer
76: acoustic lens
82: observation window
84: objective lens
86: solid-state imaging element
88: illumination window
90: cleaning nozzle
92: wiring cable
100: console
102: lesion region detection unit
104: positional information acquisition unit
106, 116: selection unit
108: lesion region detection controller
102A to 102K: first to eleventh detection units
112: organ name detection unit
114: position and orientation detection unit
118: organ name detection controller
112Ato 112K: first to eleventh detection units
140: multiplexer
142: reception circuit
144: transmission circuit
146: A/D converter
148: ASIC
150: cine memory
151: memory controller
152: CPU
154: DSC
158: pulse generation circuit
160: phase matching unit
162: B mode image generation unit
164: PW mode image generation unit
166: CF mode image generation unit
168, 168B: ultrasound image recognition unit
170: endoscope image recognition unit
172, 172B: display controller
174: color registration unit
176: organ registration unit

## Claims

1. An ultrasound endoscope system (10) comprising:
an ultrasound endoscope (12) that has an ultrasound transducer (48) at a distal end;
an ultrasound observation device (14) that makes the ultrasound transducer (48) transmit and receive an ultrasonic wave and generates an ultrasound image for diagnosis from a reception signal of the ultrasonic wave; and
an instruction acquisition unit that acquires an instruction input from a user;
**characterized in that** the ultrasound endoscope system further comprises:
an ultrasound image recognition unit (168) that learns at least one of a relationship between an ultrasound image for learning and a name of an organ displayed in the ultrasound image for learning or a relationship between the ultrasound image for learning and a position of a distal end portion of the ultrasound endoscope (12) at the time of imaging of the ultrasound image for learning, on a plurality of the ultrasound images for learning in advance, and recognizes at least one of a name of an organ displayed in the ultrasound image for diagnosis or a position of the distal end portion of the ultrasound endoscope (12) from the ultrasound image for diagnosis based on a learning result; and
a display controller (172) that displays at least one of the name of the organ or the position of the distal end portion of the ultrasound endoscope recognized by the ultrasound image recognition unit (168), on a monitor;
wherein, in response to an instruction from the user, the display controller (172) superimposedly displays the name of the organ on the ultrasound image for diagnosis and superimposedly displays the position of the distal end portion of the ultrasound endoscope (12) on an anatomical schema diagram; and
wherein, in response to an instruction from the user, the display controller (172) displays two or more images including at least one of the ultrasound image for diagnosis with the name of the organ superimposedly displayed or the anatomical schema diagram with the position of the distal end portion of the ultrasound endoscope (12) superimposedly displayed from among the ultrasound image for diagnosis with the name of the organ not displayed, the ultrasound image for diagnosis with the name of the organ superimposedly displayed, the anatomical schema diagram with the position of the distal end portion of the ultrasound endoscope (12) not displayed, and the anatomical schema diagram with the position of the distal end portion of the ultrasound endoscope superimposedly displayed, in parallel within a screen of the monitor.

2. The ultrasound endoscope system according to claim 1,
wherein the ultrasound endoscope (12) further has an illumination part and an imaging part at the distal end,
the ultrasound endoscope (12) system further comprises:
an endoscope processor (16) that makes the imaging part receive reflected light of illumination light emitted from the illumination part and generates an endoscope image for diagnosis from an imaging signal of the reflected light; wherein
the display controller (172) displays the endoscope image for diagnosis within a screen of the monitor in response to an instruction from the user, especially further comprising:
an endoscope image recognition unit (170) that recognizes a lesion region displayed in the endoscope image for diagnosis from the endoscope image for diagnosis,
wherein, in response to an instruction from the user, the display controller displays the endoscope image for diagnosis with the lesion region superimposedly displayed, on the monitor.

3. The ultrasound endoscope system according to claim 2,
wherein, in response to an instruction from the user, the display controller (172) displays two or more images including at least one of the ultrasound image for diagnosis with the name of the organ superimposedly displayed or an anatomical schema diagram with the position of the distal end portion of the ultrasound endoscope (12) superimposedly displayed from among the endoscope image for diagnosis with the lesion region not displayed, the endoscope image for diagnosis with the lesion region superimposedly displayed, the ultrasound image for diagnosis with the name of the organ not displayed, the ultrasound image for diagnosis with the name of the organ superimposedly displayed, an anatomical schema diagram with the position of the distal end portion of the ultrasound endoscope not displayed, and an anatomical schema diagram with the position of the distal end portion of the ultrasound endoscope superimposedly displayed, in parallel within the screen of the monitor.

4. The ultrasound endoscope system according to claim 1 or 3,
wherein one image of the two or more images displayed on the monitor is displayed as an image of interest to be greater than other images, especially wherein the display controller (172) switches and displays the image of interest from the one image to one of the other images in response to an instruction from the user.

5. The ultrasound endoscope system according to any one of claims 1, 3 or 4,
wherein, in response to an instruction from the user, the display controller (172) displays the ultrasound image for diagnosis with the name of the organ not displayed, the ultrasound image for diagnosis with the name of the organ superimposedly displayed, and the anatomical schema diagram with the position of the distal end portion of the ultrasound endoscope (12) superimposedly displayed, in parallel within the screen of the monitor.

6. The ultrasound endoscope system according to claim 2 or 3,
wherein the ultrasound image recognition unit (168) operates in a case where the ultrasound image for diagnosis or an anatomical schema diagram is displayed within the screen of the monitor, and the endoscope image recognition unit operates in a case where the endoscope image for diagnosis is displayed within the screen of the monitor.

7. The ultrasound endoscope system according to any one of claims 1 to 6,
wherein the ultrasound image recognition unit (168) learns at least one of the relationship between the ultrasound image for learning and the name of the organ displayed in the ultrasound image for learning or a relationship between the ultrasound image for learning and the position and an orientation of the distal end portion of the ultrasound endoscope (12) at the time of imaging of the ultrasound image for learning, on the plurality of ultrasound images for learning in advance, and recognizes at least one of the name of the organ displayed in the ultrasound image for diagnosis or the position and an orientation of the distal end portion of the ultrasound endoscope (12) from the ultrasound image for diagnosis based on a learning result, and
the display controller (172) displays at least one of the name of the organ recognized by the ultrasound image recognition unit or the position and the orientation of the distal end portion of the ultrasound endoscope (12) recognized by the ultrasound image recognition unit, on the monitor, and
especially
in response to an instruction from the user, the display controller (172) displays an anatomical schema diagram with the position and the orientation of the distal end portion of the ultrasound endoscope (12) superimposedly displayed, on the monitor.

8. The ultrasound endoscope system according to any one of claims 1 to 7,
wherein the ultrasound image recognition unit (168) further learns a relationship between the ultrasound image for learning and a range of the organ displayed in the ultrasound image for learning, on the plurality of ultrasound images for learning in advance, and recognizes the range of the organ displayed in the ultrasound image for diagnosis from the ultrasound image for diagnosis based on a learning result, and
the display controller (172) further displays the range of the organ recognized by the ultrasound image recognition unit (168), on the monitor.

9. The ultrasound endoscope system according to claim 8,
wherein the display controller (172) colors an internal region of the range of the organ recognized by the ultrasound image recognition unit (168) and displays the range of the organ with the internal region colored, on the monitor or provides a frame indicating the range of the organ recognized by the ultrasound image recognition unit (168), colors the frame, and displays the range of the organ with the frame colored, on the monitor,
especially wherein the display controller (172) colors the internal region or the frame in a different color for each type of organ with the range recognized by the ultrasound image recognition unit, and especially further comprising:
a color registration unit that registers a relationship between the type of the organ and the color of the internal region or the frame in response to an instruction from the user,
wherein the display controller (172) colors the internal region or the frame in a color designated by the instruction from the user or colors the internal region or the frame in a color of the internal region or the frame corresponding to the type of the organ registered in the color registration unit.

10. The ultrasound endoscope system according to claim 9,
wherein the ultrasound image recognition unit (168) further calculates a confidence factor of the name of the organ recognized by the ultrasound image recognition unit, and
the display controller (172) decides at least one of a display method of the name of the organ displayed on the monitor or a coloring method of the internal region or the frame depending on the confidence factor.

11. The ultrasound endoscope system according to any one of claims 9 or 10,
wherein, in a case of superimposedly displaying the name of the organ on the ultrasound image for diagnosis, the display controller (172) decides at least one of the color of the name of the organ or the color of the internal region or the frame depending on brightness of the ultrasound image for diagnosis displayed behind a display region of the name of the organ.

12. The ultrasound endoscope system according to any one of claims 9 to 11,
wherein the display controller (172) switches whether to display only one, both, or none of the name of the organ recognized by the ultrasound image recognition unit (168) and the range of the organ with the internal region or the frame colored, in response to an instruction from the user.

13. The ultrasound endoscope system according to any one of claims 8 to 12,
wherein the display controller (172) decides a position where the name of the organ recognized by the ultrasound image recognition unit (168) is displayed on the monitor, depending on the range of the organ recognized by the ultrasound image recognition unit (168).

14. The ultrasound endoscope system according to any one of claims 8 to 13,
wherein the display controller (172) decides whether or not to display the name of the organ recognized by the ultrasound image recognition unit (168) on the monitor, depending on the range of the organ recognized by the ultrasound image recognition unit (168).

15. The ultrasound endoscope system according to any one of claims 8 to 14, further comprising:
an organ registration unit that registers a type of an organ for displaying a range in response to an instruction from the user,
wherein, in a case where an organ with a range recognized by the ultrasound image recognition unit (168) is an organ registered in the organ registration unit, the display controller (172) displays the range of the organ recognized by the ultrasound image recognition unit, on the monitor.

16. The ultrasound endoscope system according to any one of claims 8 to 15,
wherein the display controller (172) sequentially switches a type of an organ for displaying a range in response to an instruction from the user.

17. The ultrasound endoscope system according to any one of claims 1 to 16,
wherein the ultrasound image recognition unit (168) is incorporated in the ultrasound observation device (14).

18. The ultrasound endoscope system according to any one of claims 1 to 16,
wherein the ultrasound image recognition unit (168) is incorporated in the endoscope processor.

19. The ultrasound endoscope system according to any one of claims 1 to 16,
wherein the ultrasound image recognition unit (168) is provided outside the ultrasound observation device and the endoscope processor.

20. A method of operating an ultrasound endoscope system (10), the method comprising:
a step of, with an ultrasound image recognition unit (168), learning at least one of a relationship between an ultrasound image for learning and a name of an organ displayed in the ultrasound image for learning or a relationship between the ultrasound image for learning and a position of a distal end portion of an ultrasound endoscope (12) at the time of imaging of the ultrasound image for learning, on a plurality of the ultrasound images for learning in advance;
a step of, with an ultrasound observation device (14), making an ultrasound transducer provided at a distal end of the ultrasound endoscope transmit and receive an ultrasonic wave and generates an ultrasound image for diagnosis from a reception signal of the ultrasonic wave;
a step of, with the ultrasound image recognition unit (168), recognizing at least one of a name of an organ displayed in the ultrasound image for diagnosis or a position of the distal end portion of the ultrasound endoscope (12) from the ultrasound image for diagnosis based on a learning result; and
a step of, with a display controller (172), displaying at least one of the name of the organ or the position of the distal end portion of the ultrasound endoscope (12) recognized by the ultrasound image recognition unit (168), on a monitor,
wherein, in response to an instruction from a user, the name of the organ is superimposedly displayed on the ultrasound image for diagnosis, and the position of the distal end portion of the ultrasound endoscope (12) is superimposedly displayed on an anatomical schema diagram, and
wherein, in response to an instruction from the user, two or more images including at least one of the ultrasound image for diagnosis with the name of the organ superimposedly displayed or the anatomical schema diagram with the position of the distal end portion of the ultrasound endoscope (12) superimposedly displayed from among the ultrasound image for diagnosis with the name of the organ not displayed, the ultrasound image for diagnosis with the name of the organ superimposedly displayed, the anatomical schema diagram with the position of the distal end portion of the ultrasound endoscope (12) not displayed, and the anatomical schema diagram with the position of the distal end portion of the ultrasound endoscope (12) superimposedly displayed are displayed in parallel within a screen of the monitor.

21. The method of operating an ultrasound endoscope system according to claim 20,
wherein one image of the two or more images displayed on the monitor is displayed as an image of interest to be greater than other images,
wherein especially the image of interest is switched from the one image to one of the other images and displayed in response to an instruction from the user.

22. The method of operating an ultrasound endoscope system according to claim 20 or 21,
wherein at least one of the relationship between the ultrasound image for learning and the name of the organ displayed in the ultrasound image for learning or a relationship between the ultrasound image for learning and the position and orientation of the distal end portion of the ultrasound endoscope (12) at the time of imaging of the ultrasound image for learning is learned on the plurality of ultrasound images for learning in advance,
at least one of the name of the organ displayed in the ultrasound image for diagnosis or the position and the orientation of the distal end portion of the ultrasound endoscope (12) is recognized from the ultrasound image for diagnosis based on a learning result, and
at least one of the name of the organ recognized by the ultrasound image recognition unit (168) or the position and the orientation of the distal end portion of the ultrasound endoscope (12) recognized by the ultrasound image recognition unit (168) is displayed on the monitor.

23. The method of operating an ultrasound endoscope system according to any one of claims 20 to 22, further comprising:
a step of, with the ultrasound image recognition unit (167), learning a relationship between the ultrasound image for learning and a range of the organ displayed in the ultrasound image for learning, on the plurality of ultrasound images for learning in advance;
a step of, with the ultrasound image recognition unit (168), recognizing the range of the organ displayed in the ultrasound image for diagnosis from the ultrasound image for diagnosis based on a learning result; and
a step of, with the display controller (172), further displaying the range of the organ recognized by the ultrasound image recognition unit (168), on the monitor.

## Patentansprüche

1. Ein Ultraschall-Endoskop-System (10), das Folgendes umfasst:
ein Ultraschall-Endoskop (12), das an einem distalen Ende einen Ultraschallwandler (48) aufweist;
eine Ultraschallbeobachtungsvorrichtung (14), die den Ultraschallwandler (48) veranlasst, eine Ultraschallwelle zu senden und zu empfangen, und aus einem Empfangssignal der Ultraschallwelle ein Ultraschallbild für die Diagnose erzeugt; und
eine Befehlserfassungseinheit, die eine Befehlseingabe von einem Benutzer erfasst;
**dadurch gekennzeichnet, dass** das Ultraschall-Endoskop-System weiterhin umfasst:
eine Ultraschallbilderkennungseinheit (168), die mindestens eine Beziehung zwischen einem Ultraschallbild zum Lernen und einem Namen eines in dem Ultraschallbild zum Lernen angezeigten Organs oder eine Beziehung zwischen dem Ultraschallbild zum Lernen und einer Position eines distalen Endabschnitts des Ultraschallendoskops (12) zum Zeitpunkt der Abbildung des Ultraschallbildes zum Lernen lernt, auf einer Vielzahl der Ultraschallbilder zum Lernen im Voraus, und erkennt mindestens einen Namen eines in dem Ultraschallbild zur Diagnose angezeigten Organs oder eine Position des distalen Endabschnitts des Ultraschallendoskops (12) aus dem Ultraschallbild zur Diagnose auf der Grundlage eines Lernergebnisses; und
eine Anzeigesteuerung (172), die auf einem Monitor den Namen des Organs und/oder die Position des von der Ultraschallbilderkennungseinheit (168) erkannten distalen Endteils des Ultraschallendoskops anzeigt;
wobei die Anzeigesteuerung (172) als Reaktion auf eine Anweisung des Benutzers den Namen des Organs auf dem Ultraschallbild zur Diagnose einblendet und die Position des distalen Endabschnitts des Ultraschall-Endoskops (12) auf einem anatomischen Schemadiagramm einblendet; und
wobei als Reaktion auf eine Anweisung des Benutzers die Anzeigesteuerung (172) zwei oder mehr Bilder anzeigt, die mindestens eines von dem Ultraschallbild für die Diagnose, bei dem der Name des Organs überlagert angezeigt wird, oder dem anatomischen Schemadiagramm, bei dem die Position des distalen Endabschnitts des Ultraschallendoskops (12) überlagert angezeigt wird, aus dem Ultraschallbild für die Diagnose, bei dem der Name des Organs nicht angezeigt wird, enthalten, dem Ultraschallbild für die Diagnose, bei dem der Name des Organs überlagert angezeigt wird, dem anatomischen Schemadiagramm, bei dem die Position des distalen Endabschnitts des Ultraschallendoskops (12) nicht angezeigt wird, und dem anatomischen Schemadiagramm, bei dem die Position des distalen Endabschnitts des Ultraschallendoskops überlagert angezeigt wird, parallel auf einem Bildschirm des Monitors.

2. Das Ultraschall-Endoskop-System nach Anspruch 1,
wobei das Ultraschall-Endoskop (12) ferner einen Beleuchtungsteil und einen Bildgebungsteil am distalen Ende aufweist,
wobei das Ultraschall-Endoskop-System (12) außerdem umfasst:
einen Endoskopprozessor (16), der dafür sorgt, dass der Bildgebungsteil reflektiertes Licht von Beleuchtungslicht empfängt, das von dem Beleuchtungsteil emittiert wird, und ein Endoskopbild zur Diagnose aus einem Bildgebungssignal des reflektierten Lichts erzeugt; wobei
die Anzeigesteuerung (172) das Endoskopbild zur Diagnose innerhalb eines Bildschirms des Monitors in Reaktion auf eine Anweisung des Benutzers anzeigt, wobei sie insbesondere weiterhin umfasst:
eine Endoskopbild-Erkennungseinheit (170), die aus dem Endoskopbild für die Diagnose einen in dem Endoskopbild angezeigten Läsionsbereich erkennt,
wobei die Anzeigesteuerung als Reaktion auf eine Anweisung des Benutzers das Endoskopbild zur Diagnose mit dem überlagert dargestellten Läsionsbereich auf dem Monitor anzeigt.

3. Das Ultraschall-Endoskop-System nach Anspruch 2,
wobei als Reaktion auf eine Anweisung des Benutzers die Anzeigesteuerung (172) zwei oder mehr Bilder anzeigt, die mindestens eines von dem Ultraschallbild für die Diagnose, bei dem der Name des Organs überlagert angezeigt wird, oder einem anatomischen Schemadiagramm, bei dem die Position des distalen Endabschnitts des Ultraschallendoskops (12) überlagert angezeigt wird, unter bzw. ausgewählt aus dem Endoskopbild für die Diagnose, bei dem der Läsionsbereich nicht angezeigt wird, dem Endoskopbild für die Diagnose, bei dem der Läsionsbereich überlagert angezeigt wird, umfassen, dem Ultraschallbild für die Diagnose mit dem nicht angezeigten Namen des Organs, dem Ultraschallbild für die Diagnose mit dem überlagert angezeigten Namen des Organs, einem anatomischen Schemadiagramm mit der Position des distalen Endabschnitts des Ultraschallendoskops, das nicht angezeigt wird, und einem anatomischen Schemadiagramm mit der Position des distalen Endabschnitts des Ultraschallendoskops, das überlagert angezeigt wird, parallel auf dem Bildschirm des Monitors.

4. Das Ultraschall-Endoskop-System nach Anspruch 1 oder 3,
wobei ein Bild der zwei oder mehr Bilder, die auf dem Monitor angezeigt werden, als ein Bild von Interesse angezeigt wird, das größer als andere Bilder ist, insbesondere wobei die Anzeigesteuerung (172) das Bild von Interesse von dem einen Bild auf eines der anderen Bilder als Reaktion auf eine Anweisung des Benutzers umschaltet und anzeigt.

5. Das Ultraschall-Endoskop-System nach einem der Ansprüche 1, 3 oder 4,
wobei als Reaktion auf eine Anweisung des Benutzers die Anzeigesteuerung (172) das Ultraschallbild für die Diagnose, bei dem der Name des Organs nicht angezeigt wird, das Ultraschallbild für die Diagnose, bei dem der Name des Organs überlagert angezeigt wird, und das anatomische Schemadiagramm, bei dem die Position des distalen Endabschnitts des Ultraschall-Endoskops (12) überlagert angezeigt wird, parallel auf dem Bildschirm des Monitors anzeigt.

6. Das Ultraschall-Endoskop-System nach Anspruch 2 oder 3,
wobei die Ultraschallbilderkennungseinheit (168) in einem Fall arbeitet, in dem das Ultraschallbild zur Diagnose oder ein anatomisches Schemadiagramm auf dem Bildschirm des Monitors angezeigt wird, und die Endoskopbilderkennungseinheit in einem Fall arbeitet, in dem das Endoskopbild zur Diagnose auf dem Bildschirm des Monitors angezeigt wird.

7. Das Ultraschall-Endoskop-System nach einem der Ansprüche 1 bis 6,
wobei die Ultraschallbilderkennungseinheit (168) mindestens eine der Beziehungen zwischen dem Ultraschallbild zum Lernen und dem Namen des in dem Ultraschallbild zum Lernen angezeigten Organs oder eine Beziehung zwischen dem Ultraschallbild zum Lernen und der Position und einer Orientierung des distalen Endabschnitts des Ultraschallendoskops (12) zum Zeitpunkt der Abbildung des Ultraschallbildes zum Lernen lernt, auf der Vielzahl von Ultraschallbildern zum Lernen im Voraus, und mindestens einen der Namen des in dem Ultraschallbild zur Diagnose angezeigten Organs oder die Position und eine Orientierung des distalen Endabschnitts des Ultraschallendoskops (12) aus dem Ultraschallbild zur Diagnose auf der Grundlage eines Lernergebnisses erkennt, und
die Anzeigesteuerung (172) mindestens einen der Namen des von der Ultraschallbilderkennungseinheit erkannten Organs oder die Position und die Ausrichtung des von der Ultraschallbilderkennungseinheit erkannten distalen Endabschnitts des Ultraschallendoskops (12) auf dem Monitor anzeigt, und
insbesondere als Reaktion auf eine Anweisung des Benutzers zeigt die Anzeigesteuerung (172) auf dem Monitor ein anatomisches Schemadiagramm an, in dem die Position und die Ausrichtung des distalen Endabschnitts des Ultraschall-Endoskops (12) überlagert dargestellt sind.

8. Das Ultraschall-Endoskop-System nach einem der Ansprüche 1 bis 7,
wobei die Ultraschallbilderkennungseinheit (168) ferner eine Beziehung zwischen dem Ultraschallbild zum Lernen und einem Bereich des in dem Ultraschallbild zum Lernen angezeigten Organs auf der Vielzahl von Ultraschallbildern zum Lernen im Voraus lernt und den Bereich des in dem Ultraschallbild zur Diagnose angezeigten Organs aus dem Ultraschallbild zur Diagnose basierend auf einem Lernergebnis erkennt, und
die Anzeigesteuerung (172) außerdem den Bereich des von der Ultraschallbilderkennungseinheit (168) erkannten Organs auf dem Monitor anzeigt.

9. Das Ultraschall-Endoskop-System nach Anspruch 8,
wobei die Anzeigesteuerung (172) einen inneren Bereich des Bereichs des von der Ultraschallbilderkennungseinheit (168) erkannten Organs einfärbt und den Bereich des Organs mit dem eingefärbten inneren Bereich auf dem Monitor anzeigt oder einen Rahmen bereitstellt, der den Bereich des von der Ultraschallbilderkennungseinheit (168) erkannten Organs anzeigt, den Rahmen einfärbt und den Bereich des Organs mit dem eingefärbten Rahmen auf dem Monitor anzeigt.
insbesondere wobei die Anzeigesteuerung (172) den inneren Bereich oder den Rahmen für jede Art von Organ mit dem von der Ultraschallbilderkennungseinheit erkannten Bereich in einer anderen Farbe einfärbt, und insbesondere weiter umfassend:
eine Farbregistrierungseinheit, die als Reaktion auf eine Anweisung des Benutzers eine Beziehung zwischen dem Typ des Organs und der Farbe des internen Bereichs oder des Rahmens registriert,
wobei die Anzeigesteuerung (172) den internen Bereich oder den Rahmen in einer Farbe einfärbt, die durch die Anweisung des Benutzers bestimmt wird, oder den internen Bereich oder den Rahmen in einer Farbe des internen Bereichs oder des Rahmens einfärbt, die dem in der Farbregistriereinheit registrierten Typ des Organs entspricht.

10. Das Ultraschall-Endoskop-System nach Anspruch 9,
wobei die Ultraschallbilderkennungseinheit (168) ferner einen Konfidenz- bzw. Vertrauensfaktor des Namens des von der Ultraschallbilderkennungseinheit erkannten Organs berechnet, und
die Anzeigesteuerung (172) in Abhängigkeit vom Konfidenzfaktor über ein Anzeigeverfahren für den Namen des auf dem Monitor angezeigten Organs oder über ein Färbungsverfahren für den internen Bereich oder den Rahmen entscheidet.

11. Das Ultraschall-Endoskop-System nach einem der Ansprüche 9 oder 10,
wobei die Anzeigesteuerung (172) in einem Fall der überlagerten Anzeige des Namens des Organs auf dem Ultraschallbild für die Diagnose mindestens eine der Farben des Namens des Organs oder der Farbe des inneren Bereichs oder des Rahmens in Abhängigkeit von der Helligkeit des Ultraschallbildes für die Diagnose, das hinter einem Anzeigebereich des Namens des Organs angezeigt wird, entscheidet.

12. Das Ultraschall-Endoskop-System nach einem der Ansprüche 9 bis 11,
wobei die Anzeigesteuerung (172) als Reaktion auf eine Anweisung des Benutzers umschaltet, ob sie nur einen, beide oder keinen der Namen des von der Ultraschallbilderkennungseinheit (168) erkannten Organs und den Bereich des Organs mit dem inneren Bereich oder dem farbigen Rahmen anzeigt.

13. Das Ultraschall-Endoskop-System nach einem der Ansprüche 8 bis 12,
wobei die Anzeigesteuerung (172) eine Position bestimmt, an der der Name des von der Ultraschallbilderkennungseinheit (168) erkannten Organs auf dem Monitor angezeigt wird, und zwar in Abhängigkeit von dem Bereich des von der Ultraschallbilderkennungseinheit (168) erkannten Organs.

14. Das Ultraschall-Endoskop-System nach einem der Ansprüche 8 bis 13,
wobei die Anzeigesteuerung (172) entscheidet, ob der Name des von der Ultraschallbilderkennungseinheit (168) erkannten Organs auf dem Monitor angezeigt wird oder nicht, abhängig von dem Bereich des von der Ultraschallbilderkennungseinheit (168) erkannten Organs.

15. Das Ultraschall-Endoskop-System nach einem der Ansprüche 8 bis 14, das außerdem Folgendes umfasst:
eine Organregistrierungseinheit, die einen Typ eines Organs zur Anzeige eines Bereichs als Reaktion auf eine Anweisung des Benutzers registriert,
wobei in einem Fall, in dem ein Organ mit einem von der Ultraschallbilderkennungseinheit (168) erkannten Bereich ein in der Organregistrierungseinheit registriertes Organ ist, die Anzeigesteuerung (172) den Bereich des von der Ultraschallbilderkennungseinheit erkannten Organs auf dem Monitor anzeigt.

16. Das Ultraschall-Endoskop-System nach einem der Ansprüche 8 bis 15,
wobei die Anzeigesteuerung (172) einen Typ eines Organs zur Anzeige eines Bereichs in Reaktion auf eine Anweisung des Benutzers sequentiell umschaltet.

17. Das Ultraschall-Endoskop-System nach einem der Ansprüche 1 bis 16,
wobei die Ultraschallbilderkennungseinheit (168) in die Ultraschallbeobachtungsvorrichtung (14) eingebaut ist.

18. Das Ultraschall-Endoskop-System nach einem der Ansprüche 1 bis 16,
wobei
die Ultraschallbilderkennungseinheit (168) in den Endoskopprozessor integriert ist.

19. Das Ultraschall-Endoskop-System nach einem der Ansprüche 1 bis 16,
wobei
die Ultraschallbilderkennungseinheit (168) außerhalb der Ultraschallbeobachtungsvorrichtung und des Endoskopprozessors vorgesehen ist.

20. Verfahren zum Betreiben eines Ultraschall-Endoskop-Systems (10), wobei das Verfahren umfasst:
einen Schritt des Lernens, mit einer Ultraschallbilderkennungseinheit (168), von mindestens einer Beziehung zwischen einem Ultraschallbild zum Lernen und einem Namen eines Organs, das in dem Ultraschallbild zum Lernen angezeigt wird, oder einer Beziehung zwischen dem Ultraschallbild zum Lernen und einer Position eines distalen Endabschnitts eines Ultraschallendoskops (12) zum Zeitpunkt der Abbildung des Ultraschallbildes zum Lernen, auf einer Vielzahl der Ultraschallbilder zum Lernen im Voraus;
einen Schritt, bei dem mit einer Ultraschallbeobachtungsvorrichtung (14) ein Ultraschallwandler, der an einem distalen Ende des Ultraschallendoskops vorgesehen ist, eine Ultraschallwelle sendet und empfängt und aus einem Empfangssignal der Ultraschallwelle ein Ultraschallbild zur Diagnose erzeugt;
einen Schritt, bei dem mit der Ultraschallbilderkennungseinheit (168) mindestens ein Name eines in dem Ultraschallbild zur Diagnose angezeigten Organs oder eine Position des distalen Endabschnitts des Ultraschallendoskops (12) aus dem Ultraschallbild zur Diagnose auf der Grundlage eines Lernergebnisses erkannt wird; und
einen Schritt, bei dem mit einer Anzeigesteuerung (172) zumindest entweder der Name des Organs oder die Position des distalen Endabschnitts des Ultraschallendoskops (12), der von der Ultraschallbilderkennungseinheit (168) erkannt wird, auf einem Monitor angezeigt wird,
wobei als Reaktion auf eine Anweisung eines Benutzers der Name des Organs auf dem Ultraschallbild zur Diagnose überlagert angezeigt wird und die Position des distalen Endabschnitts des Ultraschall-Endoskops (12) auf einem anatomischen Schemadiagramm überlagert angezeigt wird, und
wobei als Reaktion auf eine Anweisung des Benutzers zwei oder mehr Bilder, die mindestens eines von dem Ultraschallbild für die Diagnose, bei dem der Name des Organs überlagert angezeigt wird, oder dem anatomischen Schemadiagramm, bei dem die Position des distalen Endabschnitts des Ultraschall-Endoskops (12) überlagert angezeigt wird umfassen, aus dem Ultraschallbild für die Diagnose, bei dem der Name des Organs nicht angezeigt wird, angezeigt werden, dem Ultraschallbild für die Diagnose, bei dem der Name des Organs eingeblendet ist, dem anatomischen Schemadiagramm, bei dem die Position des distalen Endabschnitts des Ultraschall-Endoskops (12) nicht eingeblendet ist, und dem anatomischen Schemadiagramm, bei dem die Position des distalen Endabschnitts des Ultraschall-Endoskops (12) eingeblendet ist, parallel auf einem Bildschirm des Monitors angezeigt werden.

21. Verfahren zum Betrieb eines Ultraschall-Endoskopsystems nach Anspruch 20,
wobei ein Bild der zwei oder mehr auf dem Monitor angezeigten Bilder als ein Bild von größerem Interesse als andere Bilder angezeigt wird.
wobei insbesondere das interessierende Bild von dem einen Bild auf eines der anderen Bilder umgeschaltet und in Reaktion auf eine Anweisung des Benutzers angezeigt wird.

22. Verfahren zum Betrieb eines Ultraschall-Endoskopsystems nach Anspruch 20 oder 21,
wobei mindestens eine der Beziehungen zwischen dem Ultraschallbild zum Lernen und dem Namen des in dem Ultraschallbild zum Lernen angezeigten Organs oder eine Beziehung zwischen dem Ultraschallbild zum Lernen und der Position und Orientierung des distalen Endabschnitts des Ultraschallendoskops (12) zum Zeitpunkt der Abbildung des Ultraschallbildes zum Lernen auf der Vielzahl der Ultraschallbilder zum Lernen im Voraus gelernt wird,
mindestens einer der Namen des in dem Ultraschallbild für die Diagnose angezeigten Organs oder die Position und die Ausrichtung des distalen Endabschnitts des Ultraschallendoskops (12) aus dem Ultraschallbild für die Diagnose basierend auf einem Lernergebnis erkannt wird, und
mindestens einer der Namen des von der Ultraschallbilderkennungseinheit (168) erkannten Organs oder die Position und die Ausrichtung des von der Ultraschallbilderkennungseinheit (168) erkannten distalen Endteils des Ultraschallendoskops (12) auf dem Monitor angezeigt wird.

23. Verfahren zum Betreiben eines Ultraschall-Endoskopsystems nach einem der Ansprüche 20 bis 22, das ferner umfasst:
einen Schritt, bei dem mit der Ultraschallbilderkennungseinheit (167) eine Beziehung zwischen dem Ultraschallbild zum Lernen und einem Bereich des in dem Ultraschallbild zum Lernen angezeigten Organs auf der Vielzahl von Ultraschallbildern zum Lernen im Voraus gelernt wird;
einen Schritt, bei dem mit der Ultraschallbilderkennungseinheit (168) der Bereich des in dem Ultraschallbild für die Diagnose angezeigten Organs aus dem Ultraschallbild für die Diagnose basierend auf einem Lernergebnis erkannt wird; und
einen Schritt, in dem mit der Anzeigesteuerung (172) der Bereich des von der Ultraschallbilderkennungseinheit (168) erkannten Organs auf dem Monitor angezeigt wird.

## Revendications

1. Système d'endoscope à ultrasons (10) comprenant :
un endoscope à ultrasons (12) comportant un transducteur à ultrasons (48) à une extrémité distale ;
un dispositif d'observation des ultrasons (14) qui fait en sorte que le transducteur à ultrasons (48) transmette et reçoive une onde ultrasonore et génère une image ultrasonore pour le diagnostic à partir d'un signal de réception de l'onde ultrasonore ; et
une unité d'acquisition d'instructions qui reçoit une instruction d'un utilisateur ;
**caractérisé par le fait que** le système d'endoscope à ultrasons comprend en outre :
une unité de reconnaissance d'images ultrasonores (168) qui apprend au moins une relation entre une image ultrasonore pour l'apprentissage et un nom d'organe affiché dans l'image ultrasonore pour l'apprentissage ou une relation entre l'image ultrasonore pour l'apprentissage et une position d'une partie d'extrémité distale de l'endoscope ultrasonore (12) au moment de l'imagerie de l'image ultrasonore pour l'apprentissage, sur une pluralité d'images échographiques pour l'apprentissage à l'avance, et reconnaît au moins l'un des noms d'un organe affiché dans l'image échographique pour le diagnostic ou une position de l'extrémité distale de l'endoscope à ultrasons (12) à partir de l'image échographique pour le diagnostic sur la base d'un résultat d'apprentissage ; et
un contrôleur d'affichage (172) qui affiche sur un moniteur au moins l'un des éléments suivants : le nom de l'organe ou la position de l'extrémité distale de l'endoscope à ultrasons reconnue par l'unité de reconnaissance d'images à ultrasons (168) ;
dans lequel, en réponse à une instruction de l'utilisateur, le contrôleur d'affichage (172) affiche en surimpression le nom de l'organe sur l'image ultrasonore pour le diagnostic et affiche en surimpression la position de l'extrémité distale de l'endoscope ultrasonore (12) sur un schéma anatomique ; et
dans lequel, en réponse à une instruction de l'utilisateur, le contrôleur d'affichage (172) affiche deux images ou plus comprenant au moins l'une de l'image échographique pour le diagnostic avec le nom de l'organe affiché en surimpression ou le schéma anatomique avec la position de la partie de l'extrémité distale de l'endoscope à ultrasons (12) affichée en surimpression parmi l'image échographique pour le diagnostic avec le nom de l'organe non affiché, l'image échographique pour le diagnostic avec le nom de l'organe affiché en surimpression, le schéma anatomique avec la position de l'extrémité distale de l'endoscope à ultrasons (12) non affichée, et le schéma anatomique avec la position de l'extrémité distale de l'endoscope à ultrasons affichée en surimpression, en parallèle dans un écran du moniteur.

2. Système d'endoscope à ultrasons selon la revendication 1,
l'endoscope à ultrasons (12) comporte en outre une partie d'éclairage et une partie d'imagerie à l'extrémité distale,
le système d' endoscope à ultrasons (12) comprend en outre :
un processeur d'endoscope (16) qui fait en sorte que la partie d'imagerie reçoive la lumière réfléchie de la lumière d'illumination émise par la partie d'illumination et génère une image d'endoscope pour le diagnostic à partir d'un signal d'imagerie de la lumière réfléchie ; dans lequel
le contrôleur d'affichage (172) affiche l'image de l'endoscope pour le diagnostic dans un écran du moniteur en réponse à une instruction de l'utilisateur, comprenant en particulier :
une unité de reconnaissance d'image d'endoscope (170) qui reconnaît une région de lésion affichée dans l'image d'endoscope pour le diagnostic à partir de l'image d'endoscope pour le diagnostic,
dans lequel, en réponse à une instruction de l'utilisateur, le contrôleur d'affichage affiche sur le moniteur l'image de l'endoscope pour le diagnostic avec la région de la lésion en surimpression.

3. Système d'endoscope à ultrasons selon la revendication 2,
dans lequel, en réponse à une instruction de l'utilisateur, le contrôleur d'affichage (172) affiche deux images ou plus comprenant au moins l'une parmi l'image échographique pour le diagnostic avec le nom de l'organe affiché en surimpression ou un schéma anatomique avec la position de la partie d'extrémité distale de l'endoscope à ultrasons (12) affichée en surimpression parmi l'image endoscopique pour le diagnostic avec la région de la lésion non affichée, l'image endoscopique pour le diagnostic avec la région de la lésion affichée en surimpression, l'image échographique pour le diagnostic avec le nom de l'organe non affiché, l'image échographique pour le diagnostic avec le nom de l'organe affiché en surimpression, un schéma anatomique avec la position de l'extrémité distale de l'endoscope à ultrasons non affiché, et un schéma anatomique avec la position de l'extrémité distale de l'endoscope à ultrasons affiché en surimpression, en parallèle sur l'écran du moniteur.

4. Système d'endoscope à ultrasons selon la revendication 1 ou 3,
dans lequel une image parmi les deux images ou plus affichées sur le moniteur est affichée en tant qu'image d'intérêt plus grande que les autres images, en particulier dans lequel le contrôleur d'affichage (172) commute et affiche l'image d'intérêt de la première image à l'une des autres images en réponse à une instruction de l'utilisateur.

5. Système d'endoscope à ultrasons selon l'une des revendications 1, 3 ou 4,
dans lequel, en réponse à une instruction de l'utilisateur, le contrôleur d'affichage (172) affiche l'image échographique pour le diagnostic avec le nom de l'organe non affiché, l'image échographique pour le diagnostic avec le nom de l'organe affiché en surimpression, et le schéma anatomique avec la position de l'extrémité distale de l'endoscope à ultrasons (12) affichée en surimpression, en parallèle sur l'écran du moniteur.

6. Système d'endoscope à ultrasons selon la revendication 2 ou 3,
dans lequel l'unité de reconnaissance d'images ultrasonores (168) fonctionne dans le cas où l'image ultrasonore pour le diagnostic ou un schéma anatomique est affiché sur l'écran du moniteur, et l'unité de reconnaissance d'images d'endoscopes fonctionne dans le cas où l'image d'endoscopes pour le diagnostic est affichée sur l'écran du moniteur.

7. Système d'endoscope à ultrasons selon l'une des revendications 1 à 6,
dans lequel l'unité de reconnaissance d'images ultrasonores (168) apprend au moins l'une des relations entre l'image ultrasonore pour l'apprentissage et le nom de l'organe affiché dans l'image ultrasonore pour l'apprentissage ou une relation entre l'image ultrasonore pour l'apprentissage et la position et l'orientation de l'extrémité distale de l'endoscope ultrasonore (12) au moment de l'imagerie de l'image ultrasonore pour l'apprentissage, sur la pluralité d'images échographiques pour l'apprentissage à l'avance, et reconnaît au moins l'un des éléments suivants : le nom de l'organe affiché dans l'image échographique pour le diagnostic ou la position et l'orientation de l'extrémité distale de l'endoscope à ultrasons (12) de l'image échographique pour le diagnostic, sur la base d'un résultat d'apprentissage, et
le contrôleur d'affichage (172) affiche sur le moniteur au moins l'un des éléments suivants : le nom de l'organe reconnu par l'unité de reconnaissance d'images ultrasonores ou la position et l'orientation de l'extrémité distale de l'endoscope ultrasonore (12) reconnues par l'unité de reconnaissance d'images ultrasonores, et
en particulier en réponse à une instruction de l'utilisateur, le contrôleur d'affichage (172) affiche sur le moniteur un schéma anatomique avec la position et l'orientation de l'extrémité distale de l'endoscope à ultrasons (12) en surimpression.

8. Système d'endoscope à ultrasons selon l'une des revendications 1 à 7,
dans lequel l'unité de reconnaissance d'images ultrasonores (168) apprend en outre une relation entre l'image ultrasonore pour l'apprentissage et une plage de l'organe affiché dans l'image ultrasonore pour l'apprentissage, sur la pluralité d'images ultrasonores pour l'apprentissage à l'avance, et reconnaît la plage de l'organe affiché dans l'image ultrasonore pour le diagnostic à partir de l'image ultrasonore pour le diagnostic sur la base d'un résultat d'apprentissage, et
le contrôleur d'affichage (172) affiche en outre sur le moniteur la gamme de l'organe reconnu par l'unité de reconnaissance d'images ultrasonores (168).

9. Système d'endoscope à ultrasons selon la revendication 8,
dans lequel le contrôleur d'affichage (172) colore une région interne de la plage de l'organe reconnu par l'unité de reconnaissance d'images ultrasonores (168) et affiche la plage de l'organe avec la région interne colorée, sur le moniteur ou fournit un cadre indiquant la plage de l'organe reconnu par l'unité de reconnaissance d'images ultrasonores (168), colore le cadre, et affiche la plage de l'organe avec le cadre coloré, sur le moniteur,.
en particulier dans lequel le contrôleur d'affichage (172) colore la région interne ou le cadre d'une couleur différente pour chaque type d'organe avec la gamme reconnue par l'unité de reconnaissance d'images ultrasonores, et en particulier r comprenant :
une unité d'enregistrement des couleurs qui enregistre une relation entre le type d'organe et la couleur de la région interne ou du cadre en réponse à une instruction de l'utilisateur,
le contrôleur d'affichage (172) colore la zone interne ou le cadre dans une couleur désignée par l'instruction de l'utilisateur ou colore la zone interne ou le cadre dans une couleur de la zone interne ou du cadre correspondant au type d'orgue enregistré dans l'unité d'enregistrement des couleurs.

10. Système d'endoscope à ultrasons selon la revendication 9,
dans lequel l'unité de reconnaissance d'images ultrasonores (168) calcule en outre un facteur de confiance du nom de l'organe reconnu par l'unité de reconnaissance d'images ultrasonores, et
le contrôleur d'affichage (172) décide au moins d'une méthode d'affichage du nom de l'organe sur le moniteur ou d'une méthode de coloration de la région interne ou du cadre en fonction du facteur de confiance.

11. Système d'endoscope à ultrasons selon l'une des revendications 9 ou 10,
dans lequel, en cas de superposition du nom de l'organe sur l'image échographique pour le diagnostic, le contrôleur d'affichage (172) décide au moins de la couleur du nom de l'organe ou de la couleur de la région interne ou du cadre en fonction de la luminosité de l'image échographique pour le diagnostic affichée derrière une région d'affichage du nom de l'organe.

12. Système d'endoscope à ultrasons selon l'une des revendications 9 à 11,
dans lequel le contrôleur d'affichage (172) choisit d'afficher un seul nom de l'organe reconnu par l'unité de reconnaissance d'images ultrasonores (168) et la plage de l'organe avec la région interne ou le cadre coloré, ou les deux, ou aucun, en réponse à une instruction de l'utilisateur.

13. Système d'endoscope à ultrasons selon l'une des revendications 8 à 12,
le contrôleur d'affichage (172) décide de la position dans laquelle le nom de l'organe reconnu par l'unité de reconnaissance d'images ultrasonores (168) est affiché sur le moniteur, en fonction de la portée de l'organe reconnu par l'unité de reconnaissance d'images ultrasonores (168).

14. Système d'endoscope à ultrasons selon l'une des revendications 8 à 13,
le contrôleur d'affichage (172) décide d'afficher ou non le nom de l'organe reconnu par l'unité de reconnaissance d'images ultrasonores (168) sur le moniteur, en fonction de la portée de l'organe reconnu par l'unité de reconnaissance d'images ultrasonores (168).

15. Le système d'endoscope à ultrasons selon l'une des revendications 8 à 14, comprenant en outre :
une unité d'enregistrement d'orgue qui enregistre un type d'orgue pour l'affichage d'une gamme en réponse à une instruction de l'utilisateur,
dans lequel, dans le cas où un organe dont la portée est reconnue par l'unité de reconnaissance d'images ultrasonores (168) est un organe enregistré dans l'unité d'enregistrement d'organes, le contrôleur d'affichage (172) affiche la portée de l'organe reconnu par l'unité de reconnaissance d'images ultrasonores sur le moniteur.

16. Système d'endoscope à ultrasons selon l'une des revendications 8 à 15,
dans lequel le contrôleur d'affichage (172) change séquentiellement de type d'organe pour afficher une gamme en réponse à une instruction de l'utilisateur.

17. Système d'endoscope à ultrasons selon l'une des revendications 1 à 16,
dans lequel l'unité de reconnaissance d'images ultrasonores (168) est incorporée dans le dispositif d'observation ultrasonore (14).

18. Système d'endoscope à ultrasons selon l'une des revendications 1 à 16,
dans lequel
l'unité de reconnaissance d'images ultrasonores (168) est incorporée dans le processeur de l'endoscope.

19. Système d'endoscope à ultrasons selon l'une des revendications 1 à 16,
dans lequel
l'unité de reconnaissance d'images ultrasonores (168) est fournie à l'extérieur du dispositif d'observation ultrasonore et du processeur d'endoscope.

20. Méthode de fonctionnement d'un système d'endoscope à ultrasons (10), comprenant :
une étape consistant, à l'aide d'une unité de reconnaissance d'images ultrasonores (168), à apprendre au moins l'une des relations suivantes : une relation entre une image ultrasonore destinée à l'apprentissage et un nom d'organe affiché dans l'image ultrasonore destinée à l'apprentissage ou une relation entre l'image ultrasonore destinée à l'apprentissage et une position d'une extrémité distale d'un endoscope ultrasonore (12) au moment de l'imagerie de l'image ultrasonore destinée à l'apprentissage, sur une pluralité d'images ultrasonores destinées à l'apprentissage à l'avance ;
une étape consistant, à l'aide d'un dispositif d'observation à ultrasons (14), à faire en sorte qu'un transducteur à ultrasons situé à l'extrémité distale de l'endoscope à ultrasons transmette et reçoive une onde ultrasonore et génère une image ultrasonore pour le diagnostic à partir d'un signal de réception de l'onde ultrasonore ;
une étape de reconnaissance, par l'unité de reconnaissance d'images ultrasonores (168), d'au moins un nom d'organe affiché dans l'image ultrasonore pour le diagnostic ou une position de l'extrémité distale de l'endoscope ultrasonore (12) à partir de l'image ultrasonore pour le diagnostic, sur la base d'un résultat d'apprentissage ; et
une étape consistant, à l'aide d'un contrôleur d'affichage (172), à afficher sur un moniteur au moins l'un des éléments suivants : le nom de l'organe ou la position de l'extrémité distale de l'endoscope à ultrasons (12) reconnue par l'unité de reconnaissance d'images ultrasonores (168),
dans lequel, en réponse à une instruction d'un utilisateur, le nom de l'organe est affiché en surimpression sur l'image ultrasonore pour le diagnostic, et la position de la partie terminale distale de l'endoscope ultrasonore (12) est affichée en surimpression sur un schéma anatomique, et
dans lequel, en réponse à une instruction de l'utilisateur, deux images ou plus comprenant au moins l'une de l'image échographique pour le diagnostic avec le nom de l'organe affiché en surimpression ou le schéma anatomique avec la position de la partie de l'extrémité distale de l'endoscope à ultrasons (12) affiché en surimpression parmi l'image échographique pour le diagnostic avec le nom de l'organe non affiché, l'image échographique de diagnostic avec le nom de l'organe affiché en surimpression, le schéma anatomique avec la position de l'extrémité distale de l'endoscope à ultrasons (12) non affichée, et le schéma anatomique avec la position de l'extrémité distale de l'endoscope à ultrasons (12) affichée en surimpression sont affichés en parallèle sur l'écran du moniteur.

21. Méthode d'exploitation d'un système d'endoscope à ultrasons selon la revendication 20,
dans lequel une image parmi les deux images ou plus affichées sur le moniteur est affichée en tant qu'image d'intérêt plus important que les autres images.
dans lequel, en particulier, l'image d'intérêt passe d'une image à l'une des autres images et est affichée en réponse à une instruction de l'utilisateur.

22. Méthode d'exploitation d'un système d'endoscope à ultrasons selon la revendication 20 ou 21,
dans lequel au moins l'une des relations entre l'image ultrasonore pour l'apprentissage et le nom de l'organe affiché dans l'image ultrasonore pour l'apprentissage ou une relation entre l'image ultrasonore pour l'apprentissage et la position et l'orientation de l'extrémité distale de l'endoscope ultrasonore (12) au moment de l'imagerie de l'image ultrasonore pour l'apprentissage est apprise à l'avance sur la pluralité d'images ultrasonores pour l'apprentissage,
au moins l'un des noms de l'organe affiché dans l'image échographique pour le diagnostic ou la position et l'orientation de la partie terminale distale de l'endoscope à ultrasons (12) est reconnu à partir de l'image échographique pour le diagnostic sur la base d'un résultat d'apprentissage, et
au moins l'un des éléments suivants est affiché sur le moniteur : le nom de l'organe reconnu par l'unité de reconnaissance d'images ultrasonores (168) ou la position et l'orientation de l'extrémité distale de l'endoscope ultrasonore (12) reconnues par l'unité de reconnaissance d'images ultrasonores (168).

23. La méthode d'exploitation d'un système d'endoscope à ultrasons selon l'une des revendications 20 à 22, comprenant en outre :
une étape consistant, avec l'unité de reconnaissance d'images ultrasonores (167), à apprendre une relation entre l'image ultrasonore pour l'apprentissage et une gamme de l'organe affiché dans l'image ultrasonore pour l'apprentissage, sur la pluralité d'images ultrasonores pour l'apprentissage à l'avance ;
une étape consistant, avec l'unité de reconnaissance d'images ultrasonores (168), à reconnaître l'étendue de l'organe affiché dans l'image ultrasonore pour le diagnostic à partir de l'image ultrasonore pour le diagnostic sur la base d'un résultat d'apprentissage ; et
une étape consistant, avec le contrôleur d'affichage (172), à afficher sur le moniteur la gamme de l'organe reconnu par l'unité de reconnaissance d'images ultrasonores (168).
